(19) **Europäisches Patentamt**
**European Patent Office**
**Office européen des brevets**

(11) **EP 2 709 637 B1**

(12) **EUROPEAN PATENT SPECIFICATION**

(45) Date of publication and mention
of the grant of the patent:
**22.03.2017 Bulletin 2017/12**

(51) Int Cl.:
*A61K 38/18* (2006.01)       *A61K 38/17* (2006.01)
*A61K 35/51* (2015.01)       *A61K 35/12* (2015.01)
*A61K 35/50* (2015.01)       *A61L 27/22* (2006.01)
*A61L 27/38* (2006.01)       *A61L 27/54* (2006.01)
*C12N 5/073* (2010.01)       *A61K 35/48* (2015.01)
*A61L 27/52* (2006.01)       *A61P 29/00* (2006.01)

(21) Application number: **12725939.8**

(22) Date of filing: **17.05.2012**

(86) International application number:
**PCT/US2012/038407**

(87) International publication number:
**WO 2012/158952 (22.11.2012 Gazette 2012/47)**

(54) **TREATMENT OF INTERVERTEBRAL DISC DEGENERATION USING HUMAN UMBILICAL CORD TISSUE-DERIVED CELLS AND HYDROGEL**

BEHANDLUNG VON BANDSCHEIBENDEGENERATION ANHAND VON ZELLEN AUS MENSCHLICHEM NABELSCHNURGEWEBE UND HYDROGEL

TRAITEMENT D'UNE DÉGÉNÉRESCENCE DES DISQUES INTERVERTÉBRAUX PAR DES CELLULES ISSUES DE TISSU DE CORDON OMBILICAL HUMAIN ET D'HYDROGEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.05.2011 US 201113111933**

(43) Date of publication of application:
**26.03.2014 Bulletin 2014/13**

(73) Proprietor: **DePuy Synthes Products, LLC
Raynham, MA 02767 (US)**

(72) Inventors:
• **KRAMER, Brian C.
Plainfield
New Jersey 07060 (US)**

• **BROWN, Laura J.
Hamilton Square
New Jersey 08690 (US)**
• **KIHM, Anthony J.
Princeton
New Jersey 08540 (US)**

(74) Representative: **Hallybone, Huw George
Carpmaels & Ransford LLP
One Southampton Row
London WC1B 5HA (GB)**

(56) References cited:
**WO-A1-2009/085860       WO-A1-2009/155656
WO-A2-2006/071777       US-A1- 2006 275 273
US-A1- 2010 215 714**

SEQUENCE LISTING

<110> BROWN, LAURA
GOSIEWSKA, ANNA
KIHM, ANTHONY J.
KRAMER, BRIAN

<120> TREATMENT OF INTERVERTEBRAL DISC DEGENERATION USING HUMAN UMBILICAL CORD TISSUE-DERIVED CELLS

<130> 18668-332000 (0244C1WO)

<140>
<141> 2012-5-18

<150> 13/111,933
<151> 2011-5-19

<150> 12/337,439
<151> 2008-12-17

<150> 61/016,849
<151> 2007-12-27

<160> 10

<170> PatentIn version 3.5

<210> 1
<211> 22
<212> DNA
<213> Artificial Sequence

<220>
<223> Description of Artificial Sequence: Synthetic primer

<400> 1
gagaaatcca aagagcaaat gg          22

<210> 2
<211> 21
<212> DNA
<213> Artificial Sequence

<220>
<223> Description of Artificial Sequence: Synthetic primer

<400> 2
agaatggaaa actggaatag g          21


<210> 3
<211> 20
<212> DNA
<213> Artificial Sequence


<220>
<223> Description of Artificial Sequence: Synthetic primer


<400> 3
tcttcgatgc ttcggattcc          20


<210> 4
<211> 21
<212> DNA
<213> Artificial Sequence


<220>
<223> Description of Artificial Sequence: Synthetic primer


<400> 4
gaattctcgg aatctctgtt g          21


<210> 5
<211> 21
<212> DNA
<213> Artificial Sequence


<220>
<223> Description of Artificial Sequence: Synthetic primer


<400> 5
ttacaagcag tgcagaaaac c          21


<210> 6
<211> 22
<212> DNA
<213> Artificial Sequence


<220>
<223> Description of Artificial Sequence: Synthetic primer


<400> 6
agtaaacatt gaaaccacag cc          22


<210> 7
<211> 20
<212> DNA
<213> Artificial Sequence


<220>
<223> Description of Artificial Sequence: Synthetic primer


<400> 7
tctgcagctc tgtgtgaagg          20

<210> 8
<211> 22
<212> DNA
<213> Artificial Sequence

<220>
<223> Description of Artificial Sequence: Synthetic primer

<400> 8
cttcaaaaac ttctccacaa cc 22

<210> 9
<211> 17
<212> DNA
<213> Artificial Sequence

<220>
<223> Description of Artificial Sequence: Synthetic primer

<400> 9
cccacgccac gctctcc          17

<210> 10
<211> 19
<212> DNA
<213> Artificial Sequence

<220>
<223> Description of Artificial Sequence: Synthetic primer

<400> 10
tcctgtcagt tggtgctcc          19

**Description**

**FIELD OF THE INVENTION**

The invention relates generally to the field of cell-based therapeutics. In some aspects, the invention relates to the use of umbilical cord tissue-derived cells to treat a disease or condition related to intervertebral disc degeneration.

**BACKGROUND OF THE INVENTION**

Various publications, including patents, published applications, technical articles and scholarly articles are cited throughout the specification.

Lower back pain is one of the most common disabilities, and causes significant physical and emotional discomfort in affected individuals. Deterioration of the structure of the intervertebral disc (IVD) is one of the leading causes of lower back pain. The IVD is formed from a fibrous outer annulus fibrosus surrounding a softer, gelatinous nucleus pulposus. The fibers of the annulus fibrosus attach to the endplates of the vertebral bodies of the spinal cord and trap the nucleus pulposus, creating an isobaric environment. Under an axial load, the nucleus pulposus compresses and radially transfers that load to the annulus fibrosus. The laminated nature of the annulus fibrosus provides it with a high tensile strength and so allows it to expand radially in response to this transferred load.

In a healthy IVD, the cells within the nucleus pulposus form only about one percent of the disc tissue by volume. These cells produce an extracellular matrix (ECM) containing a high percentage of proteoglycans. The proteoglycans contain sulfated functional groups that retain water, thereby providing the nucleus pulposus with its cushioning qualities. The nucleus pulposus cells may also secrete small amounts of cytokines and matrix metalloproteinases (MMPs), which help regulate the metabolism of the nucleus pulposus cells.

In some instances of IVD disease, gradual degeneration of the IVD is caused by mechanical instabilities in other portions of the spine. In these instances, increased loads and pressures on the nucleus pulposus cause the cells within the disc (or invading macrophages) to emit larger than normal amounts of the above-mentioned cytokines. In other instances of IVD disease, genetic factors or apoptosis can cause a decline in the number of disc cells and/or a release of toxic amounts of cytokines and MMPs. In some instances, the pumping action of the disc may malfunction (due to, for example, a decrease in the proteoglycan concentration within the nucleus pulposus), thereby retarding the flow of nutrients into the disc as well as the flow of waste products out of the disc. This reduced capacity to provide nutrients to the cells and eliminate waste may result in decreased cell viability and metabolism, resulting in further degradation of the ECM along with the accumulation of high levels of toxins that may cause nerve irritation and pain.

As IVD degeneration progresses, toxic levels of cytokines and MMPs present in the nucleus pulposus begin to degrade the ECM. In particular, MMPs (as mediated by cytokines) begin cleaving the water-retaining portions of the proteoglycans, thereby reducing its water-retaining capabilities. This degradation leads to a less flexible nucleus pulposus, which changes the loading pattern within the disc, and in turn may lead to delamination of the annulus fibrosus. These changes cause more mechanical instability, which can cause the cells to emit even more cytokines and lead to upregulation of MMPs. As this destructive cascade continues and IVD degeneration progresses, the disc begins to bulge ("a herniated disc"), and then ultimately ruptures, causing the nucleus pulposus to contact the spinal cord and produce pain.

Currently, the primary therapies for IVD degeneration are surgical interventions in which degenerated discs are excised or fused with neighboring discs. Surgical therapies aim to alleviate pain and other symptoms of IVD degeneration, but do nothing to repair or regenerate diseased IVDs. One approach for treating degenerated IVD cells and tissues is the use of cell-based therapies, in which living cells are administered to repair, replace, and/or remodel diseased tissues. Several recent studies have investigated the use of cell-based therapies for degenerative IVD conditions. For example, U.S. Pat. Nos. 6,352,557 ("Ferree") and 6,340,369 ("Ferree II") teach harvesting live IVD cells from a patient, culturing the cells and transplanting them into an affected IVD. Similarly, Alini, Eur. Spine J., 2002: 11(Supp.2): S215-220, describes isolating and culturing cells from the nucleus pulposus, embedding the cells in a biomatrix, and then injecting the embedded cells into patients to restore functionality to affected IVDs. These approaches, while promising, have shown limited effectiveness in repairing degenerated IVDs and suffer from complications caused by immunological incompatibility between cell donors and recipients.

An alternative cell-based therapeutic approach is the use of stem cells, which have the ability to divide and differentiate into cells comprising diseased tissues. Transplantation of stem cells can be utilized as a clinical tool for reconstituting a target tissue, thereby restoring physiologic and anatomic functionality. The application of stem cell technology is wide-ranging, including tissue engineering, gene therapy delivery, and cell therapeutics, *i.e.,* delivery of biotherapeutic agents to a target location via exogenously supplied living cells or cellular components that produce or contain those agents (For a review, *see* Tresco, P. A. et al., Advanced Drug Delivery Reviews, 2000; 42:2-37). The identification of stem cells has stimulated research aimed at the selective generation of specific cell types for regenerative medicine. One obstacle to realization of the therapeutic potential of stem cell technology has been the difficulty of obtaining sufficient numbers of stem cells. Embryonic, or fetal tissue, is one source of stem cells. Embryonic stem and progenitor cells have been isolated from a number of mammalian species, including humans, and several such cell types have been shown capable of self-renewal and expansion, as well differentiation into a number of different cell lineages. However, the derivation of stem cells from embryonic and fetal sources has raised many ethical and moral issues that have prevented further development of embryonic stem cell therapeutics.

There is thus a need in the art for stem cell-based therapeutics which avoid the issues surrounding embryonic and fetal stem cells. Postpartum tissues, such as the umbilical cord and placenta, have generated interest as an alternative source of multipotent or pluripotent stem cells. For example, methods for recovery of stem cells by perfusion of the placenta or collection from umbilical cord blood or tissue have been described. A limitation of stem cell procurement from these methods has been an inadequate volume of cord blood or quantity of cells obtained, as well as heterogeneity in, or lack of characterization of, the populations of cells obtained from those sources.

Accordingly, a reliable, well-characterized and plentiful supply of substantially homogeneous populations of stem cells having the ability to differentiate into cells that are phenotypically similar to endogenous IVD cells would be advantageous in a variety of diagnostic and therapeutic applications for the repair, regeneration, and/or replacement of IVD cells, and for the rebuilding and/or remodeling of IVD tissues.

## SUMMARY OF THE INVENTION

In one aspect, methods are provided herein for use in the treatment of a disease or condition related to IVD degeneration (IDD). The methods comprise administering umbilical cord tissue-derived cells in an amount effective to treat the disease or condition. The umbilical cord tissue from which the cells are obtained is preferably substantially free of blood. The umbilical cord tissue-derived cells are preferably capable of self-renewal and expansion in culture and have the potential to differentiate, for example, to an IVD cell phenotype; require L-valine for growth; can grow in at least about 5% oxygen; do not produce CD117 or HLA-DR or telomerase; express alpha smooth muscle actin; and express, relative to a human fibroblast, mesenchymal stem cell, or iliac crest bone marrow cell, increased levels of interleukin 8 and reticulon 1.

In another aspect, pharmaceutical compositions are provided for use in the treatment of a disease or condition related to IVD degeneration, the compositions comprising a pharmaceutically acceptable carrier and umbilical cord tissue-derived cells in an amount effective to treat the disease or condition, wherein the umbilical cord tissue from which the cells are obtained is substantially free of blood, and wherein the cells are capable of self-renewal and expansion in culture and have the potential to differentiate, for example, to an IVD cell phenotype; require L-valine for growth; can grow in at least about 5% oxygen; do not produce CD117 or HLA-DR or telomerase; express alpha smooth muscle actin; and express, relative to a human fibroblast, mesenchymal stem cell, or iliac crest bone marrow cell, increased levels of interleukin 8 and reticulon 1.

Kits are described for treating a patient having a disease or condition related to IVD degeneration, the kits comprising instructions for using the kit in a method for treating a disease or condition related to IVD degeneration, a pharmaceutically acceptable carrier, and umbilical cord tissue-derived cells in an amount effective to treat the disease or condition, wherein the umbilical cord tissue from which the cells are obtained is substantially free of blood, and wherein the cells capable of self-renewal and expansion in culture and have the potential to differentiate, for example, to an IVD cell phenotype; require L-valine for growth; can grow in at least about 5% oxygen; do not produce CD117 or HLA-DR or telomerase; express alpha smooth muscle actin; and express, relative to a human fibroblast, mesenchymal stem cell, or iliac crest bone marrow cell, increased levels of interleukin 8 and reticulon 1. In some embodiments, kits provided herein further comprise at least one reagent and/or instructions for culturing the cells. In some embodiments, kits provided herein comprise instructions for inducing cells to at least partially differentiate *in vitro,* for example into cells displaying a nucleus pulposus cell phenotype and/or an annulus fibrosus cell phenotype.

In various embodiments, umbilical cord tissue-derived cells used in the methods, compositions, and/or kits described herein express oxidized low density lipoprotein receptor 1, reticulon, chemokine receptor ligand 3, and/or granulocyte chemotactic protein 2. In some embodiments, umbilical cord tissue-derived cells described herein express CD10, CD13, CD44, CD73, and CD90. In some embodiments, umbilical cord tissue-derived cells described herein have the ability to differentiate into annulus fibrosus and/or nucleus pulposus cells.

In various embodiments, the disease or condition related to IVD degeneration can be caused or induced by age, trauma, auto-immunity, inflammatory reaction, a genetic defect, immune-complex deposition, and/or combinations thereof. An IVD targeted for treatment can be intact or in any stage of damage or degeneration. For example, an IVD targeted for treatment may be herniated, ruptured, delaminated, and/or otherwise damaged or degenerated.

Methods provided herein comprise administration of undifferentiated umbilical tissue derived cells with a hydrogel. Human umbilical tissue derived cells produce beneficial trophic factors including but not limited to cytokines, growth factors, protease inhibitors, extracellular matrix proteins that promote survival, growth and differentiation of endogenous IVD progenitor or precursor cells. The trophic factors described here could be secreted directly by the transplanted human umbilical tissue derived cells in the host environment. Trophic factors or other cell derivatives could be collected from human umbilical tissue derived cells *ex vivo* and subsequently introduced into the patient.

In some embodiments, umbilical cord tissue-derived cells described herein are induced *in vitro* to differentiate into cells of a chondrocyte lineage, and/or into cells displaying the phenotype of an annulus fibrosus cell, a nucleus pulposus cell, and/or another IVD-like cell prior to, after, or simultaneously with administration of the cells. Accordingly, in some embodiments, methods provided herein further comprise the step of inducing umbilical cord tissue-derived cells to at least partially differentiate *in vitro*.

In some embodiments, umbilical cord tissue-derived cells may be genetically engineered to express a gene product, such as but not limited to, a gene product that promotes repair and/or regeneration of IVD tissues. For example, in some embodiments, umbilical cord tissue-derived cells are genetically engineered to express a trophic factor or other gene product. In some embodiments, the gene product exerts a trophic effect or otherwise modulates the umbilical cord tissue-derived cells, additional cell types administered with the umbilical cord tissue-derived cells, endogenous IVD cells, and/or other endogenous cells. In some embodiments, the gene product is a component of the extracellular matrix or an agent that modulates the extracellular matrix. In some embodiments, the gene product stimulates expression of one or more extracellular matrix proteins.

In some embodiments, umbilical cord tissue-derived cells are administered with at least one other cell type, such as but not limited to an annulus fibrosus cell, a nucleus pulposus cell, a fibroblast, a chondrocyte, a mesenchymal stem cell, adipose tissue derived cell, or another multipotent or pluripotent stem cell type. The at least one other cell type may be administered simultaneously with, before, or after, the umbilical cord tissue-derived cells.

In some embodiments, umbilical cord tissue-derived cells and the hydrogel are administered with at least one agent. For example, in some embodiments, umbilical cord tissue-derived cells are administered with a trophic factor, such as but not limited to, TGF-beta, GDF-5, TIMP-1, and PDGF-BB. In various embodiments, the at least one agent exerts a trophic effect on or otherwise modulates the umbilical cord tissue-derived cells, one or more additional cell types administered with the umbilical cord tissue-derived cells, endogenous IVD cells, and/or other endogenous cells. In some embodiments, the at least one agent stimulates expression of one or more extracellular matrix proteins. Other agents including but not limited to anti-inflammatory agents, cell survival agents, pain reducing agents and immunomodulatory agents. The agent may be administered simultaneously with, before, or after administration of the umbilical cord tissue-derived cells.

In various aspects, cells may be administered, directed to be administered or formulated to be administered by injection into an IVD, including, for example, the nucleus pulposus and/or the annulus fibrosus of a degenerated IVD. In some embodiments, cells are administered, directed to be administered or formulated to be administered such that the cells are encapsulated within an implantable device or by implantation of a device or matrix comprising the cells.

Another embodiment of the invention is a method of treating a disease or condition related to intervertebral disc degeneration, comprising administering a pharmaceutical composition comprising at least (1) a hydrogel and (2) an isolated homogenous population of cells obtained from human umbilical cord tissue to an intervertebral disc in an amount effective to treat the disease or condition, whereby the umbilical cord tissue is substantially free of blood, and whereby the isolated homogenous population of the cells is capable of self-renewal, expansion in culture, and has the potential to differentiate and does not express CD117 and/or telomerase. The isolated homogeneous population of cells may further have any of the following characteristics: (a) expresses reticulon, chemokine receptor ligand 3, and granulocyte chemotactic protein; (b) does not produce CD31, CD34 and HLA-DR; (c) expresses, relative to a human fibroblast, mesenchymal stem cell, or iliac crest bone marrow cell, increased levels interleukin 8 and reticulon 1; and (d) expresses CD10, CD 13, CD44, CD73, and CD90. In another embodiment of the invention, the isolated homogeneous population of cells expresses oxidized low density lipoprotein receptor 1, reticulon, chemokine receptor ligand 3, and/or granulocyte chemotactic protein. The composition may be administered by injection. In one embodiment, the composition further comprises (a) at least one other cell type, which may be engineered to express at least one exogenous gene product (such as *e.g.* a trophic factor), and/or (b) at least one agent, such as *e.g.* a trophic factor (*e.g.* TGF-beta, GDF-5, PDGF-BB and TIMP1). In another embodiment, the composition is administered into a degenerated intervertebral disc such as the nucleus pulposus or into the annulus fibrosus of the intervertebral disc. The isolated population of cell may be at least partially induced to differentiate *in vitro* prior to administration. In one embodiment of the invention, the isolated homogenous population of cells is induced to differentiate into cells displaying an annulus fibrosus cell phenotype or into cells displaying a nucleus pulposus cell phenotype.

The invention is a method of treating a disease or condition related to intervertebral disc degeneration, comprising administering a hydrogel and an isolated homogenous population of cells obtained from human umbilical cord tissue to an intervertebral disc in an amount effective to treat the disease or condition, wherein the umbilical cord tissue is substantially free of blood, and wherein the isolated homogenous population of the cells is capable of self-renewal, expansion in culture, and has potential to differentiate and does not express CD117 and/or telomerase. The isolated homogeneous population of cells may further have any of the following characteristics: (a) expresses reticulon, chemokine receptor ligand 3, and granulocyte chemotactic protein; (b) does not produce CD31, CD34 and HLA-DR; (c) expresses, relative to a human fibroblast, mesenchymal stem cell, or iliac crest bone marrow cell, increased levels of interleukin 8 and reticulon 1; and (d) express CD10, CD13, CD44, CD73, and CD90. In another embodiment, isolated homogeneous population of cells expresses oxidized low density lipoprotein receptor 1, reticulon, chemokine receptor ligand 3 and granulocyte chemotactic protein. The hydrogel and the isolated homogenous population of cells may be administered by injection. The hydrogel is administered simultaneously with, or before, or after, the isolated homogenous population of cells obtained from human umbilical cord tissue. In one embodiment, the isolated homogenous population of cells is administered within an implantable device. In another embodiment, the method further comprises administration of at least one other cell type simultaneously with, or before, or after, the isolated homogenous population of cells obtained from human umbilical cord tissue. The at least one other cell type may be engineered to express at least one exogenous gene product (such as a trophic factor or an exogenous gene product which modulates expression of one or more extracellular matrix proteins).

The method may further comprise administration of least one agent such as *e.g.* a trophic factor (*e.g.* TGF-beta, GDF-5, PDGF-BB and TIMP1) which may exert a trophic effect on the isolated homogenous cell population obtained from human umbilical cord tissue. In some embodiments, the isolated homogenous cell population and hydrogel are administered into a degenerated intervertebral disc such as the nucleus pulposus of the intervertebral disc or the annulus fibrosus of the intervertebral disc. In other embodiments, the isolated homogenous population of cells obtained from human umbilical cord tissue is cells may be induced to differentiate into cells displaying an annulus fibrosus cell phenotype or into cells displaying a nucleus pulposus cell phenotype.

Any hydrogel known in the art (such as those disclosed herein) may be used for the method. In one embodiment, the hydrogel comprises fibrinogen and thrombin. In another embodiment, the hydrogel comprises a fibrin glue such as *e.g.*, EVICEL® fibrin glue (EVICEL® Fibrin sealant (Human), Omrix Pharmaceuticals, Ltd.).

The foregoing and other features and advantages of the invention will be apparent from the following, more particular description of preferred embodiments of the invention, as illustrated in the accompanying drawings.

## BRIEF DESCRIPTION OF THE DRAWINGS

The foregoing summary, as well as the following detailed description of the invention, will be better understood when read in conjunction with the appended figures. For the purpose of illustrating the invention, the figures demonstrate embodiments of the present invention.

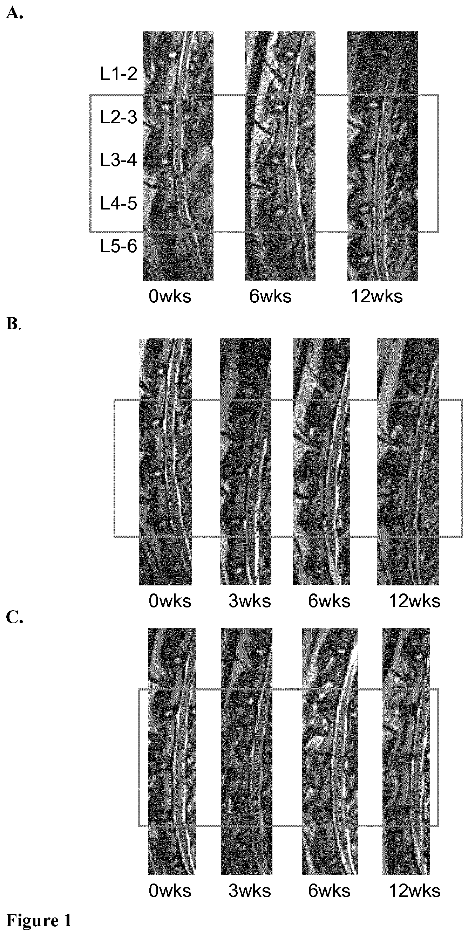

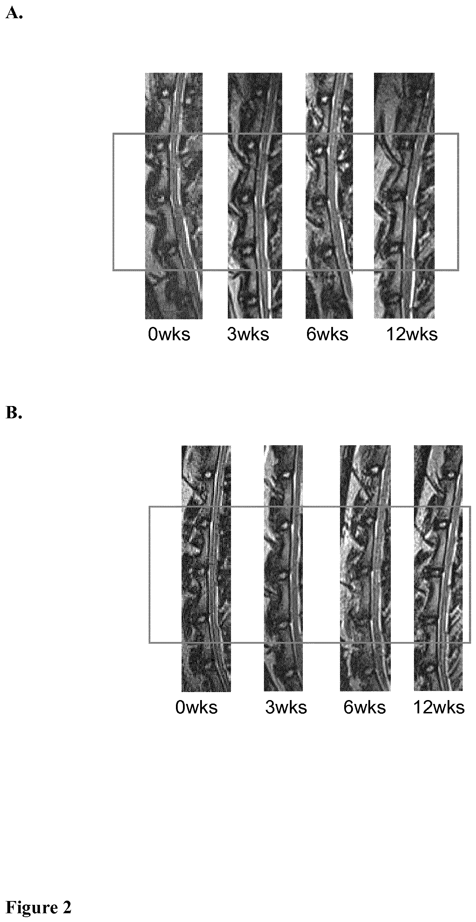

Figures 1 and 2 show lumber spine MRIs (*see* Example 12). Midsagittal T2 weighted MRIs show healthy appearing discs in the control group. The discs in the punctured group undergo degeneration (darkening and loss of height). The discs in the treated groups undergo less evidence of degeneration than the discs in the punctured group. In particular, Figure 1 and 2 show sample T2 weighted midsagittal lumbar MRI images of L1-2 through L5-6, at time points 0 (prior to annular puncture), 3 weeks (prior to injection surgery), 6 weeks, and 12 weeks (prior to sacrifice). The punctured discs (L2-3, L3-4, and L4-5) are outlined by the boxes (L2-3, L3-4, and L4-5 from top to bottom). The unpunctured control discs (L1-2 and L5-6) do not show any evidence of degeneration. The discs of the control specimen did not degenerate (Figure 1A), as expected. The punctured discs (Figure 1B) become smaller and darker across time points, suggesting degeneration. The discs that were punctured and then treated with carrier (Figure 1 C), cells + buffer (Figure 2A), and cells + carrier (Figure 2B), demonstrate less evidence of degeneration across time points compared to the punctured discs (Figure 1B).

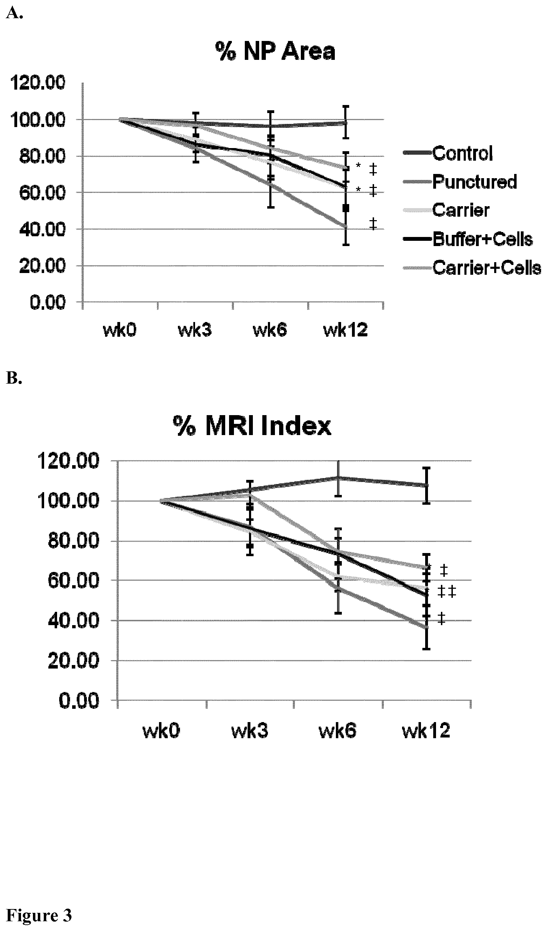

Figure 3 shows T2 Weighted MRIs (disc area and MRI index) (*see* Example 12). In particular, the average NP MRI area (Figure 3A) and MRI index (Figure 3B) combining L2-3, L3-4, and L4-5 (the treated discs) for each rabbit group, expressed as a percent of the time 0 value, demonstrates that the punctured group undergoes the largest decrease in area and index across time points, while the groups that were punctured and subsequently treated with carrier, cells in buffer (B+C), or cells in carrier (C+C) undergo a smaller decrease in area and index. In Figure 3, ‡ indicates significance compared to control and * indicates significance compared to puncture. Also, the MRI index in Figure 3 is determined as follows:

$$\text{MRI Index} = \text{NP Area x Signal Intensity}$$

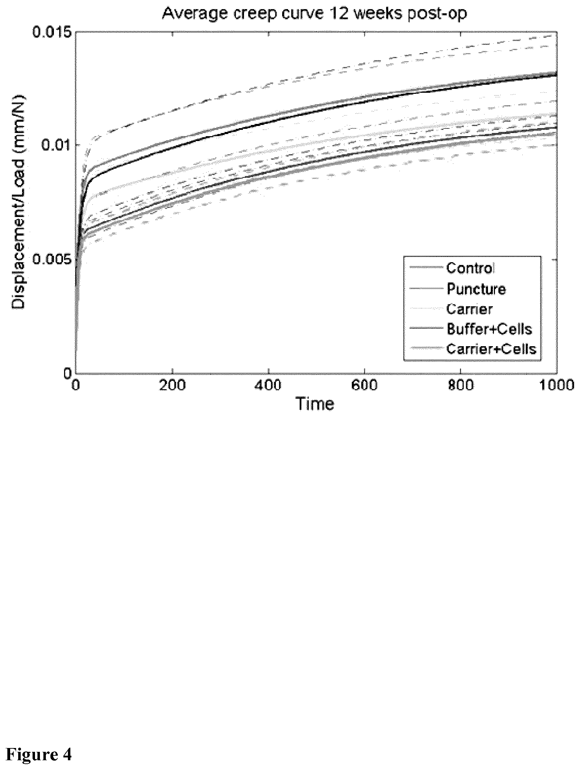

Figure 4 shows the average normalized total displacement (ramp phase + creep) curves of potted L3-4 FSU after 12 weeks (*see* Example 12). Time dependent displacement under constant load generates distinct appearing creep curves. Buffer + cells behaves more like punctured, carrier + cells behaves more like control, carrier alone falls somewhere in between. Average creep curves were generated for each condition. Axial testing generates distinct appearing creep curves in the early phase of testing (time 0 to 200 seconds). Dotted boundaries represent standard error of measurement. The curves generated for puncture and buffer + cells appear similar, as do curves for control and carrier + cells. Each of these groups appears distinct from the curve generated for the carrier group.

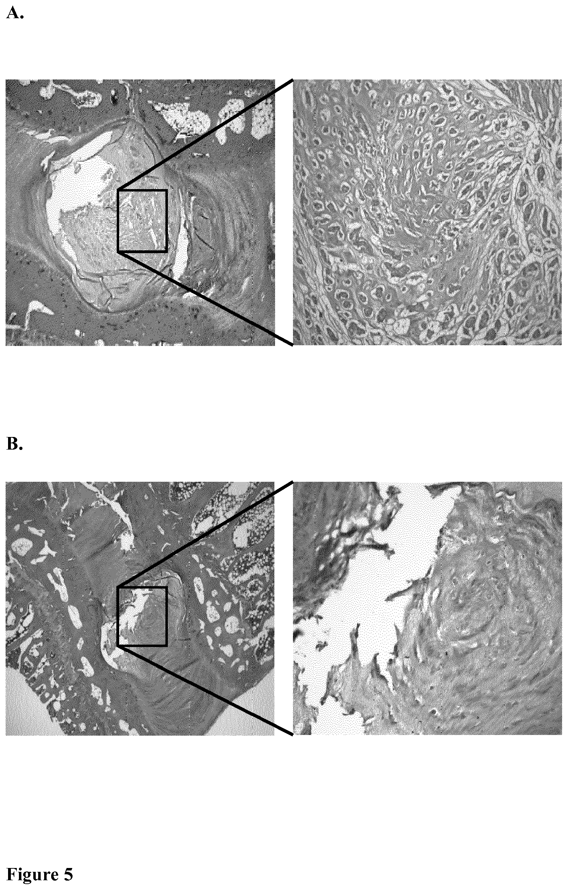

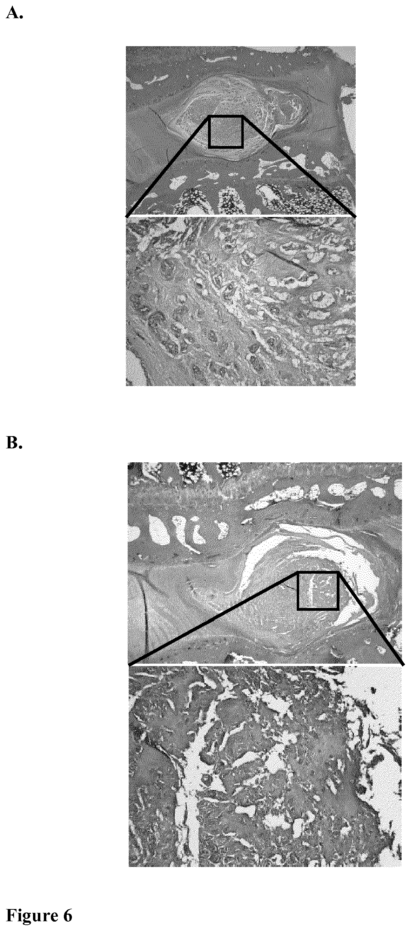

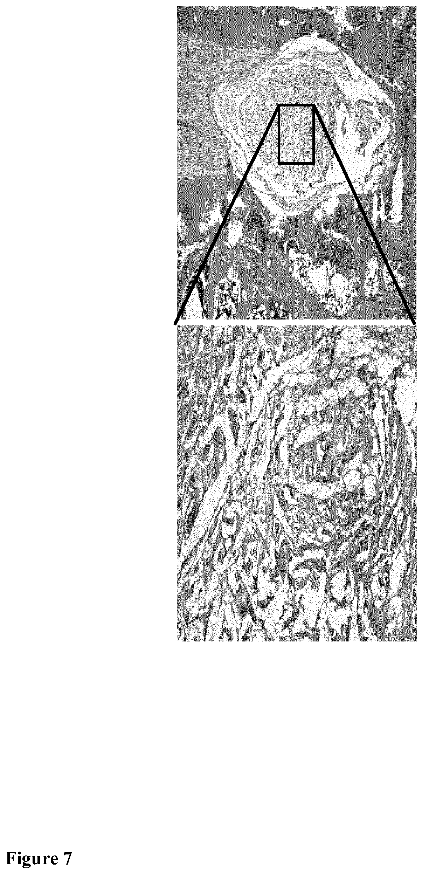

Figures 5, 6 and 7 show histology sagittal slices of disc L4-5 obtained after sacrifice at 12 weeks for each treatment group, stained with H&E, magnified 20x and 100x (*see* Example 12). Figure 5A shows the histology sagittal slice of disc L4-5 for the control group. Figure 5B shows the histology sagittal slice of disc L4-5 for the puncture group. Figure 6A shows the histology sagittal slice of disc L4-5 for the carrier group. Figure 6B shows the histology sagittal slice of disc L4-5 for the buffer and cells group. Figure 7 shows the histology sagittal slice of disc L4-5 for the carrier and cells group.

## DETAILED DESCRIPTION OF ILLUSTRATIVE EMBODIMENTS

Various terms relating to the methods and other aspects of the present invention are used throughout the specification and claims. Such terms are to be given their ordinary meaning in the art unless otherwise indicated. Other specifically defined terms are to be construed in a manner consistent with the definition provided herein.

As used in this specification and the appended claims, the singular forms "a," "an," and "the" include plural referents unless the content clearly dictates otherwise. Thus, for example, reference to "a cell" includes a combination of two or more cells, and the like.

The term "about" as used herein when referring to a measurable value such as an amount, a temporal duration, and the like, is meant to encompass variations of $\pm$ 20% or $\pm$ 10%, more preferably $\pm$ 5%, even more preferably $\pm$ 1%, and still more preferably $\pm$ 0.1% from the specified value, as such variations are appropriate to perform the disclosed methods.

"Derived" is used to indicate that the cells have been obtained from their biological source and grown, expanded in culture, immortalized, or otherwise manipulated *in vitro.*

"Isolated" means altered "by the hand of man" from the natural state. If a molecule or composition occurs in nature, it has been "isolated" if it has been changed or removed from its original environment, or both.

The term "express," "expressed," or "expression" of a nucleic acid molecule or gene refers to the biosynthesis of a gene product, for example, the biosynthesis of a polypeptide.

"Trophic factors" are substances that promote survival, growth, differentiation, proliferation and/or maturation of a cell, or stimulate increased biological activity of a cell. Cell derivatives refer to any material that can be obtained from cells and include cell conditioned media, cell lysate, extracellular matrix proteins, trophic factors, cell fractions, cell membranes.

"Degeneration" refers to any physical harm, injury, degeneration, or trauma to an IVD.

"Pathology" refers to any structural or functional indicia of a deviation from the normal state of a cell, tissue, organ, or system, as measured by any means suitable in the art.

A "disease" is any deviation from or impairment in the health, condition, or functioning of a cell, tissue, organ, system, or organism on the whole, as measured by any means suitable in the art.

"Treat," treating" or "treatment" refer to any success or indicia of success in the attenuation or amelioration of disease, damage, or condition, including any objective or subjective parameter such as abatement, remission, diminishing of symptoms or making the disease, damage, or condition more tolerable to the patient, slowing in the rate of degeneration or decline, making the final point of degeneration less debilitating, or improving a subject's physical or mental well-being. The treatment or amelioration of symptoms can be based on objective or subjective parameters; including the results of a physical examination, neurological examination, and/or psychiatric evaluations.

"Effective amount" or "therapeutically effective amount" are used interchangeably herein, and refer to an amount of a compound, material, or composition, as described herein effective to achieve a particular biological result such as, but not limited to, biological results disclosed, described, or exemplified herein. Such results may include, but are not limited to, the treatment of IVD disease or damage in a subject, as determined by any means suitable in the art.

"Pharmaceutically acceptable" refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability. "Pharmaceutically acceptable carrier" refers to a medium that does not interfere with the effectiveness of the biological activity of the active ingredient(s) and is not toxic to the host to which it is administered.

It has been discovered that diseases and conditions related to intervertebral disc (IVD) degeneration can be treated by administering umbilical cord tissue-derived cells and a hydrogel as described herein. Advantageously, methods, compositions, and kits provided herein promote repair and regeneration of degenerated IVDs, and thereby alleviate one or more symptoms associated with IVD degeneration. Accordingly, in one aspect, methods are provided for treating a disease or condition related to IVD degeneration, comprising administering umbilical cord tissue-derived cells to an IVD in an amount sufficient to treat the disease or condition.

In various embodiments, the disease or condition related to IVD degeneration can be caused or induced by age, trauma, auto-immunity, inflammatory reaction, a genetic defect, immune-complex deposition (*e.g.*, formation of scar tissue), and/or combinations thereof. An IVD targeted for treatment can be intact or in any stage of damage or degeneration. For example, an IVD targeted for treatment may be herniated (*e.g.,* wherein a portion of the annulus fibrosus has a bulge or other protrusion), ruptured (*e.g.,* wherein at least a portion of the annulus fibrosus is ruptured, resulting in a decrease in the pressure and/or volume of the nucleus pulposus), delaminated (*e.g.,* wherein two or more layers of the annulus fibrosus have separated), and/or otherwise damaged or degenerated (*e.g.,* wherein the annulus fibrosus has fissures, cracks, tears, or the like, and/or wherein the extracellular matrix is degraded or altered).

In various embodiments, umbilical cord tissue-derived cells are administered to a degenerated IVD, for example by injection, transplanting, implanting, injecting, or providing as a matrix-cell complex, or any other means known in the art for providing cell therapy. In some embodiments, cells are administered directly to the annulus fibrosus and/or the nucleus pulposus of the IVD. In some embodiments, cells are administered to an IVD indirectly. For example, cells may be in an aqueous carrier, encapsulated in a device or seeded in a matrix, which is then implanted in or near a degenerated IVD. Aqueous carriers include, but are not limited to physiological buffer solutions such as buffered saline, phosphate buffered saline, Hank's balanced salts solution, Tris buffered saline, and Hepes buffered saline. In various embodiments, a device, matrix, or other cellular depot may be implanted so that it is attached to an outer wall of the annulus fibrosus, or located outside of, but adjacent to a wall of the annulus fibrosis, or adjacent to an endplate of a vertebral body surrounding the IVD.

In some embodiments, the cells are administered in the form of a device such as a matrix-cell complex. Device materials include but are not limited to bioresorbable materials such as collagens, 35/65 Poly(epsilon-caprolactone)(PCL)/Poly(glycolic acid) (PGA), PANACRYL™ bioabsorbable constructs, VICRYL™ polyglactin 910, and self-assembling peptides and non-resorbable materials such as fluoropolymers (*e.g.,* TEFLON® fluoropolymers), plastic, and metal. Matrices include biocompatible scaffolds, lattices, self-assembling structures and the like, whether bioabsorbable or not, liquid, gel, or solid. Such matrices are known in the art of therapeutic cell treatment, surgical repair, tissue engineering, and wound healing. Preferably, the matrices are pretreated with the therapeutic cells. More preferably, the matrices are populated with cells in close association to the matrix or its spaces. The cells can adhere to the matrix or can be entrapped or contained within the matrix spaces. Most preferred are matrix-cell complexes in which the cells are growing in close association with the matrix and when used therapeutically, growth, repair, and/or regeneration of the patient's own IVD cells is stimulated and supported, and proper angiogenesis is similarly stimulated or supported. The matrix-cell compositions can be introduced into a patient's body in any way known in the art, including but not limited to implantation, injection, surgical attachment, transplantation with other tissue, and the like. In some embodiments, the matrices form *in vivo,* or even more preferably *in situ,* for example *in situ* polymerizable gels can be used in accordance with the invention. Examples of such gels are known in the art.

Umbilical cord tissue-derived cells are administered to a degenerated IVD as part of a composition comprising a hydrogel. In another embodiment, the umbilical cord tissue-derived cells are administered to a degenerated IVD along with a hydrogel.

Cells described herein can also be seeded onto three-dimensional matrices, such as scaffolds and implanted *in vivo,* where the seeded cells may proliferate on or in the framework, or help to establish replacement tissue *in vivo* with or without cooperation of other cells. Growth of umbilical cord tissue-derived cells on the three-dimensional framework preferably results in the formation of a three-dimensional tissue, or foundation thereof, which can be utilized *in vivo,* for example to repair and/or regenerate damaged or diseased tissue.

The cells can be seeded on a three-dimensional framework or matrix, such as a scaffold, a foam, an electrostatically spun scaffold, a non-woven scaffold, a porous or nonporous microparticulate, or hydrogel and administered accordingly. The framework can be configured into various shapes such as substantially flat, substantially cylindrical or tubular, or can be completely free-form as may be required or desired for the corrective structure under consideration. Two or more substantially flat frameworks can be laid atop another and secured together as necessary to generate a multilayer framework.

On such three-dimensional frameworks, the cells can be co-administered with other cell types, or other soft tissue type progenitors, including stem cells. When grown in a three-dimensional system, the proliferating cells can mature and segregate properly to form components of adult tissues analogous to counterparts found naturally *in vivo.*

The matrices described and exemplified herein can be designed such that the matrix structure supports the umbilical cord tissue-derived cells without subsequent degradation, supports the cells from the time of seeding until the tissue transplant is remodeled by the host tissue, or allows the seeded cells to attach, proliferate, and develop into a tissue structure having sufficient mechanical integrity to support itself *in vitro,* at which point, the matrix is degraded.

The matrices, scaffolds, such as foams non-wovens, electrostatically spun structures, microparticulate and self-assembling systems contemplated for use herein can be implanted in combination with any one or more cells, growth factors, drugs, or other components, such as bioactive agents that promote healing, regeneration, repair, or in-growth of tissue, or stimulate vascularization or innervation thereof or otherwise enhance or improve the therapeutic outcome or the practice of the invention, in addition to the cells of the invention.

In some embodiments, cells described herein can be grown freely in culture, removed from the culture and inoculated onto a three-dimensional framework. Inoculation of the three-dimensional framework with a concentration of cells, e.g., approximately $10^6$ to $5 \times 10^7$ cells per milliliter, preferably results in the establishment of the three-dimensional support in relatively shorter periods of time. Moreover, in some application it may be preferable to use a greater or lesser number of cells depending on the result desired.

In some aspects, it is useful to re-create in culture the cellular microenvironment found *in vivo,* such that the extent to which the cells are grown prior to implantation *in vivo* or used *in vitro* may vary. The cells can be inoculated onto the framework before or after forming the shape desired for implantation, *e.g.,* ropes, tubes, filaments, and the like. Following inoculation of the cells onto the framework, the framework is preferably incubated in an appropriate growth medium. During the incubation period, the inoculated cells will grow and envelop the framework and may for example bridge, or partially bridge any interstitial spaces therein. It is preferable, but not required to grow the cells to an appropriate degree, which reflects the *in vivo* cell density of the tissue being repaired or regenerated. In other embodiments, the presence of the cells, even in low numbers on the framework encourages in-growth of endogenous healthy cells to facilitate healing for example of the damaged or injured tissue.

Examples of matrices, for example scaffolds, which may be used include mats, porous or semiporous foams, self-assembling peptides and the like. Nonwoven mats may, for example, be formed using fibers comprised of natural or synthetic polymers. In a preferred embodiment, absorbable copolymers of glycolic and lactic acids (PGA/PLA), sold under the trade name VICRYL™ (Ethicon, Inc., Somerville, NJ) are used to form a mat. Foams, composed of, for example, poly(epsilon-caprolactone)/poly(glycolic acid) (PCL/PGA) copolymer, formed by processes such as freeze-drying, or lyophilization, as discussed in U.S. Patent No. 6,355,699, can also serve as scaffolds.

Gels are used herein. Examples of gels include injectable gels, *in situ* polymerizable gels, and hydrogels; gels may be composed of self-assembling peptides. These materials are frequently used as supports for growth of tissue. For example, when used as an injectable gel, a gel may be comprised of water, saline or physiological buffer solution and a gelling material. Gelling materials include, but are not limited to proteins such as, collagen, elastin, thrombin, fibronectin, gelatin, fibrin, tropoelastin, polypeptides, laminin, proteoglycans, fibrin glue, fibrin clot, platelet rich plasma (PRP) clot, platelet poor plasma (PPP) clot, self-assembling peptide hydrogels, and atelocollagen; polysaccharides such as, pectin, cellulose, oxidized cellulose, chitin, chitosan, agarose, hyaluronic acid; polynucleotides such as, ribonucleic acids, deoxyribonucleic acids, and others such as, alginate, cross-linked alginate, poly(N- isopropylacrylamide), poly(oxyalkylene), copolymers of poly(ethylene oxide)-poly(propylene oxide), poly(vinyl alcohol), polyacrylate, monostearoyl glycerol co-Succinate/polyethylene glycol (MGSA/PEG) copolymers and combinations thereof. In one embodiment, the gelling material (*i.e.* the hydrogel) comprises fibrinogen (factor I), such as *e.g.* recombinant fibrinogen or fibrinogen purified from blood. In another embodiment the hydrogel comprises fibrinogen and thrombin. In yet another embodiment, the gel is EVICEL® fibrin glue (EVICEL® Fibrin sealant (Human), Omrix Pharmaceuticals, Ltd.) (BAC2 (fibrinogen) and thrombin).

In general, hydrogels are cross-linked polymeric materials that can absorb more than 20% of their weight in water while maintaining a distinct three-dimensional structure. In addition, hydrogels have high permeability for oxygen, nutrients and other water-soluble metabolites. This definition includes dry cross-linked polymers that will swell in aqueous environments, as well as water-swollen materials. A host of hydrophilic polymers can be cross-linked to produce hydrogels, whether the polymer is of biological origin, semisynthetic, or wholly synthetic. The hydrogel may be produced from a synthetic polymeric material. Such synthetic polymers can be tailored to a range of properties and predictable lotto-lot uniformity, and represent a reliable source of material that generally is free from concerns of immunogenicity. In one embodiment of the invention, the hydrogels are formed from self-assembling peptides, as those discussed in U.S. Patent Nos. 5,670,483 and 5,955,343, U.S. Patent Publication No. 2002/0160471, PCT Application No. WO 02/062969.

Properties that make hydrogels particularly valuable as a matrix in this invention include the equilibrium swelling degree, sorption kinetics, solute permeability, and their *in vivo* performance characteristics. Permeability to compounds depends in part upon the swelling degree or water content and the rate of biodegradation. Since the mechanical strength of a gel declines in direct proportion to the swelling degree, it is also well within the contemplation of the present invention that the hydrogel can be attached to a substrate so that the composite system enhances mechanical strength. In alternative embodiments, the hydrogel can be impregnated within a porous substrate, to gain the mechanical strength of the substrate, along with the useful delivery properties of the hydrogel.

*In situ*-forming degradable networks are also suitable for use in the invention (see, *e.g.,* Anseth, K.S. et al., J. Controlled Release, 2002; 78:199-209; Wang, D. et al., Biomaterials, 2003; 24:3969-3980; U.S. Patent Publication 2002/0022676 to He et al.). These materials are formulated as fluids suitable for injection, and then may be induced by a variety of means (*e.g.,* change in temperature, pH, exposure to light) to form degradable hydrogel networks *in situ* or *in vivo.*

The framework can be a felt, which can be comprised of a multifilament yarn made from a bioabsorbable material, *e.g.,* PGA, PLA, PCL copolymers or blends, or hyaluronic acid. The yarn is made into a felt using standard textile processing techniques consisting of crimping, cutting, carding and needling. The cells of the invention can be seeded onto foam scaffolds that may be composite structures. In addition, the three-dimensional framework may be molded into a useful shape, such as a specific structure in or around the IVD to be repaired, replaced, or augmented.

The framework can be treated prior to inoculation of the cells of the invention in order to enhance cell attachment. For example, prior to inoculation with the cells of the invention, nylon matrices could be treated with 0.1 molar acetic acid and incubated in polylysine, PBS, and/or collagen to coat the nylon. Polystyrene could be similarly treated using sulfuric acid.

In addition, the external surfaces of the three-dimensional framework can be modified to improve the attachment or growth of cells and differentiation of tissue, such as by plasma coating the framework or addition of one or more proteins (*e.g.,* collagens, elastic fibers, reticular fibers), glycoproteins, glycosaminoglycans (*e.g.,* heparin sulfate, chondroitin-4-sulfate, chondroitin-6-sulfate, dermatan sulfate, keratin sulfate), a cellular matrix, and/or other materials such as, but not limited to, gelatin, alginates, agar, agarose, and plant gums, among others.

The scaffold can be comprised of or treated with materials that render it non-thrombogenic. These treatments and materials may also promote and sustain endothelial growth, migration, and extracellular matrix deposition. Examples of these materials and treatments include but are not limited to natural materials such as basement membrane proteins such as laminin and Type IV collagen, synthetic materials such as ePTFE, and segmented polyurethaneurea silicones, such as PURSPAN® (The Polymer Technology Group, Inc., Berkeley, CA). These materials can be further treated to render the scaffold non-thrombogenic. Such treatments include anti-thrombotic agents such as heparin, and treatments which alter the surface charge of the material such as plasma coating.

Different proportions of the various types of collagen, for example, deposited on the framework can affect the growth of tissue-specific or other cells which may be later inoculated onto the framework or which may grow onto the structure *in vivo.* Alternatively, the framework can be inoculated with a mixture of cells which synthesize the appropriate collagen types desired. Depending upon the tissue to be cultured, the appropriate collagen type to be inoculated on the framework or produced by the cells seeded thereon may be selected. For example, the relative amounts of collagenic and elastic fibers present in the framework can be modulated by controlling the ratio of collagen-producing cells to elastin-producing cells in the initial inoculum.

The seeded or inoculated three-dimensional framework can be for transplantation or implantation of either the cultured cells obtained from the matrix or the cultured matrix itself *in vivo.* The three-dimensional scaffolds may be used to replace or augment existing tissue, to introduce new or altered tissue, to modify artificial prostheses, or to join together biological tissues or structures.

The cells may be administered (*e.g.,* injected) into an IVD through a needle, such as a small bore needle. In some embodiments, the needle has a bore of about 22 gauge or less, so as to mitigate the possibility of herniating the IVD. When injecting volumes into the nucleus pulposus, it is desirable that the volume of drug delivered be no more than about 3 ml, preferably no more than about 1 ml, more preferably between about 0.1 and about 0.5 ml. When injected in these smaller quantities, it is believed the added volume will not cause an appreciable pressure increase in the nucleus pulposus. If the volume of the direct injection of the formulation is sufficiently high so as to cause a concern of overpressurizing the nucleus pulposus, then it is preferred that at least a portion of the nucleus pulposus be removed prior to direct injection. In some embodiments, the volume of removed nucleus pulposus is substantially similar to the volume of the formulation to be injected. For example, the volume of removed nucleus pulposus can be within about 80-120% of the volume of the formulation to be injected. In some embodiments, the umbilical cord tissue-derived cells are concentrated prior to being administered.

Cells useful in methods, compositions, and kits provided herein can be derived from mammalian umbilical cord recovered upon or shortly after termination of either a full-term or pre-term pregnancy, for example, following expulsion after birth or surgical removal following a Cesarean section. Blood and debris are removed from the umbilical cord tissue prior to isolation of cells, for example, by washing with any suitable medium or buffer.

Cells can be isolated from umbilical cord tissue by mechanical force or by enzymatic digestion. Preferred enzymes are metalloproteases, neutral proteases and mucolytic proteases. For example, various combinations of collagenase, dispase, and hyaluronidase can be used to dissociate cells from the umbilical cord tissue. The skilled artisan will appreciate that many such enzyme treatments are known in the art for isolating cells from various tissue sources. For example, the LIBERASE® Blendzyme (Roche) series of enzyme combinations are suitable for use in the instant methods. Other sources of enzymes are known, and the skilled artisan may also obtain such enzymes directly from their natural sources. The skilled artisan is also well-equipped to assess new, or additional enzymes or enzyme combinations for their utility in isolating the cells of the invention. Preferred enzyme treatments are 0.5, 1, 1.5, or 2 hours long or longer.

Isolated cells can be used to initiate cell cultures. Isolated cells are transferred to sterile tissue culture vessels either uncoated or coated with extracellular matrix or ligands such as laminin, collagen (native, denatured, atello, or crosslinked), gelatin, fibronectin, and other extracellular matrix proteins. Umbilical cord tissue-derived cells are cultured in any culture medium capable of sustaining growth of the cells such as, but not limited to, DMEM (high or low glucose), advanced DMEM, DMEM/MCDB 201, Eagle's basal medium, Ham's F10 medium (F10), Ham's F-12 medium (F12), Hayflick's Medium, Iscove's modified Dulbecco's medium, Mesenchymal Stem Cell Growth Medium (MSCGM), DMEM/F12, RPMI 1640, and CELL-GRO-FREE. The culture medium can be supplemented with one or more components including, for example, fetal bovine serum, preferably about 2-15% (v/v); equine serum; human serum; fetal calf serum; beta-mercaptoethanol, preferably about 0.001% (v/v); one or more growth factors, for example, platelet-derived growth factor (PDGF), epidermal growth factor (EGF), fibroblast growth factor (FGF), vascular endothelial growth factor (VEGF), insulin-like growth factor-1 (IGF-1), leukocyte inhibitory factor (LIF) and erythropoietin; amino acids, including L-valine; and one or more antibiotic and/or antimycotic agents to control microbial contamination, such as, for example, penicillin G, streptomycin sulfate, amphotericin B, gentamicin, and nystatin, either alone or in combination.

The cells are seeded in culture vessels at a density to allow cell growth. In one embodiment, the cells are cultured at about 0 to about 5 percent by volume $CO_2$ in air. In some embodiments, the cells are cultured at about 2 to about 25 percent $O_2$ in air, preferably about 5 to about 20 percent $O_2$ in air. The cells preferably are cultured at about 25 to about 40°C and more preferably are cultured at 37°C. The medium in the culture vessel can be static or agitated, for example, using a bioreactor. Umbilical cord tissue-derived cells are preferably grown under low oxidative stress (*e.g.,* with addition of glutathione, Vitamin C, Catalase, Vitamin E, N-Acetylcysteine), meaning no or minimal free radical damage to the cultured cells.

Umbilical cord tissue-derived cells can be passaged, or removed to a separate culture vessel containing fresh medium of the same or a different type as that used initially, where the population of cells can be mitotically expanded. The cells of the invention may be used at any point between passage 0 and senescence. The cells preferably are passaged between about 3 and about 25 times, more preferably are passaged about 4 to about 12 times, and preferably are passaged 10 or 11 times. Cloning and/or subcloning may be performed to confirm that a clonal population of cells has been isolated.

In one embodiment of the invention, the cells may be grown (expanded) on a microcarrier. Microcarriers are particles, beads or pellets useful for attachment and growth of anchorage dependent cells in culture. Microcarriers may be solid, porous or a solid core with a porous coating. Exemplary suitable microcarriers include but are not limited to Cytodex 1®, Cytodex 2®, Cytodex 3® (GE Healthcare Life Sciences, Piscataway NJ) or HILLEX® (SoloHill Engineering, Inc. Ann Arbor, MI). Exemplary suitable microcarriers and microcarrier components are disclosed in U.S. Patent Publication No. 2008/016632 8.

Different cell types present in umbilical cord tissue can be fractionated into subpopulations. This may be accomplished using standard techniques for cell separation including, but not limited to, enzymatic treatment; cloning and selection of specific cell types, for example but not limited to selection based on morphological and/or biochemical markers; selective growth of desired cells (positive selection), selective destruction of unwanted cells (negative selection); separation based upon differential cell agglutinability in the mixed population as, for example, with soybean agglutinin; freeze-thaw procedures; differential adherence properties of the cells in the mixed population; filtration; conventional and zonal centrifugation; centrifugal elutriation (counter-streaming centrifugation); unit gravity separation; countercurrent distribution; electrophoresis; fluorescence activated cell sorting (FACS); and the like.

Examples of cells isolated from umbilical cord tissue were deposited with the American Type Culture Collection on June 10, 2004, and assigned ATCC Accession Numbers as follows: (1) strain designation UMB 022803 (P7) was assigned Accession No. PTA-6067; and (2) strain designation UMB 022803 (P17) was assigned Accession No. PTA-6068.

Umbilical cord tissue-derived cells can be characterized by, for example, by growth characteristics (*e.g.,* population doubling capability, doubling time, passages to senescence), karyotype analysis (*e.g.,* normal karyotype; maternal or neonatal lineage), flow cytometry (*e.g.*, FACS analysis), immunohistochemistry and/or immunocytochemistry (*e.g.*, for detection of epitopes), gene expression profiling (*e.g.,* gene chip arrays; polymerase chain reaction (for example, reverse transcriptase PCR, real time PCR, and conventional PCR)), protein arrays, protein secretion (*e.g.,* by plasma clotting assay or analysis of PDC-conditioned medium, for example, by Enzyme Linked ImmunoSorbent Assay (ELISA)), mixed lymphocyte reaction (*e.g.,* as measure of stimulation of PBMCs), and/or other methods known in the art.

In various aspects, the umbilical cord tissue-derived cells have one or more of the following growth features: require L-valine for growth in culture; are capable of growth in atmospheres containing oxygen from about 5% to at least about 20%; have the potential for at least about 40 doublings in culture before reaching senescence; and/or attach and expand on a coated or uncoated tissue culture vessel, wherein the coated tissue culture vessel comprises a coating of gelatin, laminin, collagen, polyornithine, vitronectin or fibronectin.

In some embodiments, the cells have a normal karyotype, which is maintained as the cells are passaged. Karyotyping is particularly useful for identifying and distinguishing neonatal from maternal cells derived from placenta. Methods for karyotyping are available and known to those of skill in the art.

In some embodiments, the cells can be characterized by production of certain proteins, including production of at least one of tissue factor, vimentin, and alpha-smooth muscle actin; and production of at least one of CD10, CD13, CD44, CD73, CD90, PDGFr-alpha, PD-L2 and HLA-A,B,C cell surface markers, as detected by, for example, flow cytometry. In other embodiments, the cells may be characterized by lack of production of at least one ofCD31, CD34, CD45, CD80, CD86, CD117, CD141, CD178, B7-H2, HLA-G, and HLA-DR, HLA-DP, and/or HLA-DQ cell surface markers, as detected by any suitable means, such as flow cytometry. In some embodiments, cells that produce at least two of tissue factor, vimentin, and alpha-smooth muscle actin are preferred. In some embodiments, cells producing all three of the proteins tissue factor, vimentin, and alpha-smooth muscle actin are preferred.

In some embodiments, the cells have, relative to a human cell that is a fibroblast, a mesenchymal stem cell, or an iliac crest bone marrow cell, increased expression of a gene encoding at least one of interleukin 8; reticulon 1; chemokine (C-X-C motif) ligand 1 (melanoma growth stimulating activity, alpha); chemokine (C-X-C motif) ligand 6 (granulocyte chemotactic protein 2); chemokine (C-X-C motif) ligand 3; tumor necrosis factor, alpha-induced protein 3.

In yet other embodiments, the cells have, relative to a human cell that is a fibroblast, a mesenchymal stem cell, or an iliac crest bone marrow cell, reduced expression of a gene encoding at least one of: short stature homeobox 2; heat shock 27 kDa protein 2; chemokine (C-X-C motif) ligand 12 (stromal cell-derived factor 1); elastin (supravalvular aortic stenosis, Williams-Beuren syndrome); *Homo sapiens* mRNA; cDNA DKFZp586M2022 (from clone DKFZp586M2022); mesenchyme homeo box 2 (growth arrest-specific homeo box); sine oculis homeobox homolog 1 (*Drosophila*); crystallin, alpha B; disheveled associated activator of morphogenesis 2; DKFZP586B2420 protein; similar to neuralin 1; tetranectin (plasminogen binding protein); src homology three (SH3) and cysteine rich domain; cholesterol 25-hydroxylase; runt-related transcription factor 3; interleukin 11 receptor, alpha; procollagen C-endopeptidase enhancer; frizzled homolog 7 (*Drosophila*); hypothetical gene BC008967; collagen, type VIII, alpha 1; tenascin C (hexabrachion); iroquois homeobox protein 5; hephaestin; integrin, beta 8; synaptic vesicle glycoprotein 2; neuroblastoma, suppression of tumorigenicity 1; insulin-like growth factor binding protein 2, 36kDa; *Homo sapiens* cDNA FLJ12280 fis, clone MAMMA1001744; cytokine receptor-like factor 1; potassium intermediate/small conductance calcium-activated channel, subfamily N, member 4; integrin, beta 7; transcriptional co-activator with PDZ-binding motif (TAZ); sine oculis homeobox homolog 2 (Drosophila); KIAA1034 protein; vesicle-associated membrane protein 5 (myobrevin); EGF-containing fibulin-like extracellular matrix protein 1; early growth response 3; distal-less homeo box 5; hypothetical protein FLJ20373; aldo-keto reductase family 1, member C3 (3-alpha hydroxysteroid dehydrogenase, type II); biglycan; transcriptional co-activator with PDZ-binding motif (TAZ); fibronectin 1; proenkephalin; integrin, beta-like 1 (with EGF-like repeat domains); *Homo sapiens* mRNA full length insert cDNA clone EUROIMAGE 1968422; EphA3; KIAA0367 protein; natriuretic peptide receptor C/guanylate cyclase C (atrionatriuretic peptide receptor C); hypothetical protein FLJ14054; Homo sapiens mRNA; cDNA DKFZp564B222 (from clone DKFZp564B222); BCL2/adenovirus E1B 19kDa interacting protein 3-like; AE binding protein 1; and cytochrome c oxidase subunit VIIa polypeptide 1 (muscle).

In some embodiments, the cells can be characterized by secretion of at least one of MCP-1, IL-6, IL-8, GCP-2, HGF, KGF, FGF, HB-EGF, BDNF, TPO, MIP1b, RANTES, and TIMP1. In some embodiments, the cells can be characterized by lack of secretion of at least one of TGF-beta2, ANG2, PDGFbb, MIP1a and VEGF, as detected by ELISA.

In some preferred embodiments, the cell comprises two or more of the above-listed growth, protein/surface marker production, gene expression or substance-secretion characteristics. In some embodiments, cells comprising, three, four, or five or more of the characteristics are preferred. In some embodiments, cells comprising six, seven, or eight or more of the characteristics are preferred. In some embodiments, cells comprising all of above characteristics are preferred. In other embodiments, the umbilical-derived cells have any of the characteristics disclosed in U.S. Patent Nos. 7,510,873 and 7,524,4 89.

Among cells that are preferred for use with the various aspects of the invention are cells having the characteristics described above, and more particularly those wherein the cells have normal karyotypes and maintain normal karyotypes with passaging, and further wherein the cells express each of the markers CD10, CD13, CD44, CD73, CD90, PDGFr-alpha, and HLA-A,B,C, wherein the cells produce the immunologically-detectable proteins which correspond to the listed markers. Also preferred are those cells which, in addition to the foregoing, do not produce proteins corresponding to any of the markers CD31, CD34, CD45, CD117, CD141, or HLA-DR,DP,DQ, as detected by any means suitable in the art, such as flow cytometry. Highly preferred are cells that do not express CD117 or HLA-DR or telomerase.

In some preferred aspects, methods comprise administering a hydrogel and cells obtained or isolated from human umbilical cord tissue to a degenerated IVD, wherein the cells are capable of self-renewal and expansion in culture, require L-valine for growth, can grow in at least about 5% oxygen, do not produce CD117 or HLA-DR or telomerase, express alpha smooth muscle actin, and express, relative to a human fibroblast, mesenchymal stem cell, or iliac crest bone marrow cell, increased levels of interleukin 8 and reticulon 1. Cells isolated from human umbilical cord tissue may be expanded in culture prior to administration. In some embodiments, the cells obtained from human umbilical cord tissue have the potential to differentiate into cells of an IVD phenotype, such as but not limited to, an annulus fibrosus cell phenotype or a nucleus pulposus cell phenotype. The umbilical cord tissue-derived cells can integrate into the patient's IVD, or alternatively can provide support for growth or stimulation to differentiate for naturally present IVD stem cells. The survival of the administered cells is not determinative of the success or results of their use where there is improvement in the disease or condition related to IVD degeneration and/or overall patient health. In some embodiments, the cells preferably at least partially integrate, multiply, or survive in the patient. In some embodiments, the patient experiences benefits from the therapy, for example from the ability of the cells to support the growth of other cells, including stem cells or progenitor cells present in the IVD and/or surrounding tissues, from the tissue in-growth or vascularization of the tissue, and/or from the presence of beneficial cellular factors, chemokines, cytokines and the like, but the cells do not integrate or multiply in the patient. In some aspects, the patient benefits from the therapeutic treatment with the cells, but the cells do not survive for a prolonged period in the patient. For example, in some embodiments, the cells gradually decline in number, viability or biochemical activity. In some embodiments, such a decline may be preceded by a period of activity, for example growth, division, or biochemical activity. In some embodiments, senescent, nonviable or even dead cells are able to have a beneficial therapeutic effect.

Certain cells having the potential to differentiate along lines leading to various phenotypes are unstable and thus can spontaneously differentiate. Thus, in some embodiments, cells that do not spontaneously differentiate are preferred. For example, some preferred cells, when grown in Growth Medium, are substantially stable with respect to the cell markers produced on their surface, and with respect to the expression pattern of various genes, for example as determined using gene expression profiling using, for example, nucleic acid or polypeptide arrays. Such cells remain substantially constant, for example in their surface marker characteristics, upon passaging, through multiple population doublings.

In some embodiments, methods provided herein induce umbilical cord tissue-derived cells to differentiate along an IVD cell pathway, towards IVD cell phenotypes, or progenitors or more primitive relatives of the foregoing. The umbilical cord tissue-derived cells can integrate into the patient's IVD, or alternatively can provide support for growth or stimulation to differentiate for naturally present IVD stem cells. The survival of the administered cells is not determinative of the success or results of their use where there is improvement in the disease or condition related to IVD degeneration and/or overall patient health. In some embodiments, the cells preferably at least partially integrate, multiply, or survive in the patient. In some embodiments, the patient experiences benefits from the therapy, for example from the ability of the cells to support the growth of other cells, including stem cells or progenitor cells present in the IVD and/or surrounding tissues, from the tissue in-growth or vascularization of the tissue, and/or from the presence of beneficial cellular factors, chemokines, cytokines and the like, but the cells do not integrate or multiply in the patient. In some aspects, the patient benefits from the therapeutic treatment with the cells, but the cells do not survive for a prolonged period in the patient. For example, in some embodiments, the cells gradually decline in number, viability or biochemical activity. In some embodiments, such a decline may be preceded by a period of activity, for example growth, division, or biochemical activity. In some embodiments, senescent, nonviable or even dead cells are able to have a beneficial therapeutic effect.

The methods further comprise evaluating the patient for improvements in IVD structure and/or function, and/or improvements in overall health. Such evaluations can proceed according to any means suitable in the art, including those described and exemplified herein.

In some embodiments, umbilical cord tissue-derived cells are administered in conjunction with one or more other cell types, including but not limited to, other multipotent or pluripotent cells, or chondrocytes, chondroblasts, osteocytes, osteoblasts, osteoclasts, bone lining cells, or bone marrow cells. The cells of different types may be admixed with the umbilical cord tissue-derived cells immediately or shortly prior to administration, or they may be co-cultured together for a period of time prior to administration. In some embodiments, a population of umbilical cord tissue-derived cells supports the survival, proliferation, growth, maintenance, maturation, differentiation, and/or increased activity of one or more other cell types administered in conjunction with the umbilical cord tissue-derived cells, and/or vice versa.

Umbilical cord tissue-derived cells provide trophic support to other cell types with which they are administered, and/or vice versa. In some embodiments, it is desirable for the umbilical cord tissue-derived cells and the co-cultured cells to be in contact. This can be achieved, for example, by seeding the cells as a heterogeneous population of cells in culture medium or onto a suitable culture substrate. Alternatively, the umbilical cord tissue-derived cells can first be grown to confluence and employed as a substrate for the co-cultured cells. In other embodiments, the co-cultured cells may be cultured in contact with a conditioned medium, extracellular matrix, and/or cell lysate of the umbilical cord tissue-derived cells.

Umbilical cord tissue-derived cells can be administered in conjunction with a biologically active agent, such as an agent that modulates proliferation, differentiation, and/or other cellular activities. The agent can be administered before, after, or simultaneously as the umbilical cord tissue-derived cells. The particular agent chosen can be at the discretion of the medical professional directing the treatment of the patient, and can vary according to the particular needs or condition of the patient. The agent chosen can be used for various purposes such as, but not limited to, facilitating the administration of the cells, improving the repair and/or regeneration of the IVD, improving the overall health of the patient, reducing pain, reducing or preventing rejection of the transplanted cells, and the like.

Examples of agents that may be administered with umbilical cord tissue-derived cells include, but are not limited to, vitamins and other nutritional supplements; antithrombogenic agents; anti-apoptotic agents; anti-inflammatory agents; immunosuppressants (*e.g.,* cyclosporine, rapamycin); antioxidants; hormones; glycoproteins; fibronectin; peptides and proteins; carbohydrates (simple and/or complex); proteoglycans; oligonucleotides (sense and/or antisense DNA and/or RNA); bone morphogenetic proteins (BMPs); differentiation factors; antibodies (for example, antibodies to infectious agents, tumors, drugs or hormones); and gene therapy reagents. In some embodiments, the agent is a substance, such as an analgesic, anti-inflammatory, narcotic, muscle relaxer, or combination thereof that alleviates one or more symptoms of a disease or condition related to IVD degeneration.

In some embodiments, the additional agent is a trophic factor or other agent that exerts a trophic effect against the umbilical cord tissue-derived cells, against additional cells administered with the umbilical cord tissue-derived cells, against endogenous IVD cells (*e.g.*, annulus fibrosus cells, nucleus pulposus cells), and/or against other endogenous cells (*e.g.*, connective tissue progenitor cells). In some embodiments, the trophic factor is one that is secreted by umbilical cord tissue-derived cells, in which case it can be derived from preparations of such umbilical cord tissue-derived cells or from another source. Examples of such factors or agents include, but are not limited to, members of the fibroblast growth factor family, including acidic and basic fibroblast growth factor (FGF-1 and FGF-2) and FGF-4; members of the platelet-derived growth factor (PDGF) family, including PDGF-AB, PDGF-BB and PDGF-AA; EGFs, members of the insulin-like growth factor (IGF) family, including IGF-I and -II; the TGF-beta superfamily, including TGF-beta1, 2 and 3 (including MP-52), osteoid-inducing factor (OIF), angiogenin(s), endothelins, hepatocyte growth factor and keratinocyte growth factor; members of the bone morphogenetic protein (BMP) family, such as BMP-1, BMP-3, BMP-2, OP-1, BMP-2A, BMP-2B, BMP-4, BMP-7 and BMP-14; HBGF-1 and HBGF-2; growth differentiation factors (GDFs); members of the hedgehog family of proteins, including indian, sonic and desert hedgehog; ADMP-1; GDF-5; TIMP-1 and members of the colony-stimulating factor (CSF) family, including CSF-1, G-CSF, and GM-CSF; and analogues and isoforms thereof.

In some embodiments, the growth factor is selected from the group consisting of TGF-beta, TGF-beta1, FGF, bFGF, and IGF-1. These growth factors are believed to promote regeneration of the nucleus pulposus, stimulate proliferation and/or differentiation of chondrocytes, and promote extracellular matrix secretion. In some embodiments, the growth factor is TGF-beta. More preferably, TGF-beta is administered in an amount of between about 10 ng/ml and about 5000 ng/ml, for example, between about 50 ng/ml and about 500 ng/ml, *e.g.,* between about 100 ng/ml and about 300 ng/ml.

In some embodiments, platelet concentrate is provided as an additional therapeutic agent. In some embodiments, the platelet concentrate is autologous. In some embodiments, the platelet concentrate is platelet rich plasma (PRP). PRP is advantageous because it contains growth factors that can restimulate the growth of the ECM, and because its fibrin matrix provides a suitable scaffold for new tissue growth. In some embodiments, the additional agent is a cell lysate, a soluble cell fraction, a membrane-enriched cell fraction, cell culture media (*e.g.,* conditioned media), or extracellular matrix derived from umbilical cord tissue-derived cells or other cells.

Umbilical cord tissue-derived cells can be administered in conjunction with an HMG-CoA reductase inhibitor, including but not limited to simvastatin, pravastatin, lovastatin, fluvastatin, cerivastatin, and atorvastatin.

Umbilical cord tissue-derived cells can be genetically engineered to express one or more agents, such as but not limited to, one or more of the additional therapeutic agents described herein. The cells of the invention can be engineered using any of a variety of vectors including, but not limited to, integrating viral vectors, *e.g.,* retrovirus vector or adeno-associated viral vectors; non-integrating replicating vectors, *e.g.,* papilloma virus vectors, SV40 vectors, adenoviral vectors; or replication-defective viral vectors. Other methods of introducing DNA into cells include the use of liposomes, electroporation, a particle gun, or by direct DNA injection.

Hosts cells are preferably transformed or transfected with DNA controlled by or in operative association with, one or more appropriate expression control elements such as promoter or enhancer sequences, transcription terminators, polyadenylation sites, among others, and a selectable marker.

Following the introduction of the foreign DNA, engineered cells may be allowed to grow in enriched media and then switched to selective media. The selectable marker in the foreign DNA confers resistance to the selection and allows cells to stably integrate the foreign DNA as, for example, on a plasmid, into their chromosomes and grow to form foci, which, in turn, can be cloned and expanded into cell lines. This method can be advantageously used to engineer cell lines, which express the gene product.

Any promoter may be used to drive the expression of the inserted gene. For example, viral promoters include, but are not limited to, the CMV promoter/enhancer, SV40, papillomavirus, Epstein-Barr virus or elastin gene promoter. Preferably, the control elements used to control expression of the gene of interest should allow for the regulated expression of the gene so that the product is synthesized only when needed *in vivo.* If transient expression is desired, constitutive promoters are preferably used in a non-integrating and/or replication-defective vector. Alternatively, inducible promoters could be used to drive the expression of the inserted gene when necessary. Inducible promoters include, but are not limited to, those associated with metallothionein and heat shock proteins.

The cells of the invention may be genetically engineered to "knock out" or "knock down" expression of factors that promote inflammation or rejection at the implant site. Negative modulatory techniques for the reduction of target gene expression levels or target gene product activity levels are discussed below. "Negative modulation," as used herein, refers to a reduction in the level and/or activity of target gene product relative to the level and/or activity of the target gene product in the absence of the modulatory treatment. The expression of a gene can be reduced or knocked out using a number of techniques including, for example, inhibition of expression by inactivating the gene completely (commonly termed "knockout") using the homologous recombination technique. Usually, an exon encoding an important region of the protein (or an exon 5' to that region) is interrupted by a positive selectable marker, *e.g.,* neo, preventing the production of normal mRNA from the target gene and resulting in inactivation of the gene. A gene may also be inactivated by creating a deletion in part of a gene, or by deleting the entire gene. By using a construct with two regions of homology to the target gene that are far apart in the genome, the sequences intervening the two regions can be deleted (Mombaerts et al., Proc. Nat. Acad. Sci. U.S.A., 1991; 88:3084-3087).

Antisense, small interfering RNA, DNAzymes and ribozyme molecules which inhibit expression of the target gene can also be used in accordance with the invention to reduce the level of target gene activity. For example, antisense RNA molecules, which inhibit the expression of major histocompatibility gene complexes (HLA), have been shown to be most versatile with respect to immune responses. Still further, triple helix molecules can be utilized in reducing the level of target gene activity.

These and other techniques are described in detail by L. G. Davis et al. (eds), Basic Methods In Molecular Biology, 2nd ed., Appleton & Lange, Norwalk, Conn. (1994).

IL-1 is a potent stimulator of cartilage resorption and of the production of inflammatory mediators by chondrocytes (Campbell et al., J. Immun., 1991; 147(4):1238-1246). Using any of the foregoing techniques, the expression of IL-1 can be knocked out or knocked down in the cells of the invention to reduce the risk of resorption of implanted cartilage or the production of inflammatory mediators by the cells of the invention. Likewise, the expression of MHC class II molecules can be knocked out or knocked down in order to reduce the risk of rejection of the implanted tissue.

Once the cells of the invention have been genetically engineered, they may be directly implanted with the hydrogel into the patient to allow for the treatment of a disease or condition related to IVD degeneration, for example by producing a product having a therapeutic effect against one or more symptoms of the disease or condition, such as an anti-inflammatory gene product. Alternatively, the genetically engineered cells may be used to produce new tissue *in vitro,* which is then implanted in the subject, as described herein.

In some aspects, pharmaceutical compositions are provided that comprise umbilical cord tissue-derived cells, as described herein, and a pharmaceutically acceptable carrier. Pharmaceutical compositions provided herein may induce umbilical cord tissue-derived cells to differentiate along an IVD cell pathway or lineage, for example to display a nucleus pulposus cell phenotype and/or an annulus fibrosus cell phenotype. In some embodiments, pharmaceutical compositions provided herein modulate cellular processes of endogenous IVD cells and/or cells of surrounding tissues, including but not limited to, cell division, differentiation, and gene expression. In some embodiments, pharmaceutical compositions provided herein promote repair and regeneration of a degenerated IVD.

Kits for treating a patient having a disease of or damage to at least one IVD are provided. The kits comprise a pharmaceutically acceptable carrier, cells obtained from human umbilical cord tissue in an amount effective to treat the disease or condition, such as those cells that are described and exemplified herein, and instructions for using the kit in a method for treating a patient having a disease of or condition related to IVD degeneration. The kits may further comprise at least one reagent and instructions for culturing the cells. The kits may further comprise a population of at least one other cell type, and/or at least one agent.

In some aspects, the kits comprise a pharmaceutically acceptable carrier, a lysate, extracellular matrix, or conditioned medium prepared from cells obtained from human umbilical cord tissue, which cells have the characteristics that are described and exemplified herein. The kits have utility to facilitate the repair and/or regeneration of an IVD that is damaged or diseased.

The following examples are provided to describe the invention in greater detail. They are intended to illustrate, not to limit, the invention. Additionally as used in the following examples and elsewhere in the specification, the umbilical cord tissue-derived cells useful in the methods of the invention may be isolated and characterized according to the disclosure of U.S. Patent Nos. 7,510,873 and 7,5 24489.

### EXAMPLE 1

### Isolation of Umbilical Cord Tissue-Derived Cells

Umbilical cords were obtained from National Disease Research Interchange (NDRI, Philadelphia, PA). The tissues were obtained following normal deliveries. The cell isolation protocol was performed aseptically in a laminar flow hood. To remove blood and debris, the cord was washed in phosphate buffered saline (PBS; Invitrogen, Carlsbad, CA) in the presence of antimycotic and antibiotic (100 units/milliliter penicillin, 100 micrograms/milliliter streptomycin, 0.25 micrograms/milliliter amphotericin B). The tissues were then mechanically dissociated in 150 $cm^2$ tissue culture plates in the presence of 50 milliliters of medium (DMEM-Low glucose or DMEM-High glucose; Invitrogen), until the tissue was minced into a fine pulp. The chopped tissues were transferred to 50 milliliter conical tubes (approximately 5 grams of tissue per tube). The tissue was then digested in either DMEM-Low glucose medium or DMEM-High glucose medium, each containing antimycotic and antibiotic as described above. In some experiments, an enzyme mixture of collagenase and dispase was used ("C:D;" collagenase (Sigma, St Louis, MO), 500 Units/milliliter; and dispase (Invitrogen), 50 Units/milliliter in DMEM:-Low glucose medium). In other experiments a mixture of collagenase, dispase and hyaluronidase ("C:D:H") was used (collagenase, 500 Units/milliliter; dispase, 50 Units/milliliter; and hyaluronidase (Sigma), 5 Units/milliliter, in DMEM:-Low glucose). The conical tubes containing the tissue, medium and digestion enzymes were incubated at 37°C in an orbital shaker (Environ, Brooklyn, NY) at 225 rpm for 2 hours.

After digestion, the tissues were centrifuged at 150 x g for 5 minutes, the supernatant was aspirated. The pellet was resuspended in 20 milliliters of Growth Medium (DMEM: Low glucose (Invitrogen), 15 percent (v/v) fetal bovine serum (FBS; defined bovine serum; Lot#AND18475; Hyclone, Logan, UT), 0.001% (v/v) 2-mercaptoethanol (Sigma), 1 milliliter per 100 milliliters of antibiotic/antimycotic as described above. The cell suspension was filtered through a 70-micrometer nylon cell strainer (BD Biosciences). An additional 5 milliliters rinse comprising Growth Medium was passed through the strainer. The cell suspension was then passed through a 40-micrometer nylon cell strainer (BD Biosciences) and chased with a rinse of an additional 5 milliliters of Growth Medium.

The filtrate was resuspended in Growth Medium (total volume 50 milliliters) and centrifuged at 150 x g for 5 minutes. The supernatant was aspirated and the cells were resuspended in 50 milliliters of fresh Growth Medium. This process was repeated twice more.

Upon the final centrifugation supernatant was aspirated and the cell pellet was resuspended in 5 milliliters of fresh Growth Medium. The number of viable cells was determined using Trypan Blue staining. Cells were then cultured under standard conditions.

The cells isolated from umbilical cords were seeded at 5,000 cells/cm$^2$ onto gelatin-coated T-75 cm$^2$ flasks (Corning Inc., Corning, NY) in Growth Medium with antibiotics/antimycotics as described above. After 2 days (in various experiments, cells were incubated from 2-4 days), spent medium was aspirated from the flasks. Cells were washed with PBS three times to remove debris and blood-derived cells. Cells were then replenished with Growth Medium and allowed to grow to confluence (about 10 days from passage 0) to passage 1. On subsequent passages (from passage 1 to 2 and so on), cells reached sub-confluence (75-85 percent confluence) in 4-5 days. For these subsequent passages, cells were seeded at 5000 cells/cm$^2$. Cells were grown in a humidified incubator with 5 percent carbon dioxide and atmospheric oxygen, at 37°C.

## EXAMPLE 2

### Evaluation of Human Postpartum-Derived Cell Surface Markers by Flow Cytometry

Umbilical cord tissue was characterized using flow cytometry to provide a profile for the identification of cells obtained therefrom.

Cells were cultured in Growth Medium (Gibco Carlsbad, CA) with penicillin/streptomycin. Cells were cultured in plasma-treated T75, T150, and T225 tissue culture flasks (Corning, Corning, NY) until confluent. The growth surfaces of the flasks were coated with gelatin by incubating 2% (w/v) gelatin (Sigma, St. Louis, MO) for 20 minutes at room temperature.

Adherent cells in flasks were washed in PBS and detached with Trypsin/EDTA. Cells were harvested, centrifuged, and resuspended in 3% (v/v) FBS in PBS at a cell concentration of 1x10$^7$ per milliliter. In accordance to the manufacture's specifications, antibody to the cell surface marker of interest (see below) was added to one hundred microliters of cell suspension and the mixture was incubated in the dark for 30 minutes at 4 °C. After incubation, cells were washed with PBS and centrifuged to remove unbound antibody. Cells were resuspended in 500 microliter PBS and analyzed by flow cytometry. Flow cytometry analysis was performed with a FACScalibur instrument (Becton Dickinson, San Jose, CA).

The following antibodies to cell surface markers were used:

| Antibody | Manufacture | Catalog Number |
|---|---|---|
| CD10 | BD Pharmingen (San Diego, CA) | 555375 |
| CD13 | BD Pharmingen | 555394 |
| CD31 | BD Pharmingen | 555446 |
| CD34 | BD Pharmingen | 555821 |
| CD44 | BD Pharmingen | 555478 |
| CD45RA | BD Pharmingen | 555489 |
| CD73 | BD Pharmingen | 550257 |
| CD90 | BD Pharmingen | 555596 |
| CD117 | BD Pharmingen | 340529 |
| CD141 | BD Pharmingen | 559781 |
| PDGFr-alpha | BD Pharmingen | 556002 |
| HLA-A, B, C | BD Pharmingen | 555553 |

(continued)

| Antibody | Manufacture | Catalog Number |
| --- | --- | --- |
| HLA-DR, DP, DQ | BD Pharmingen, | 555558 |
| IgG-FITC | Sigma (St. Louis, MO) | F-6522 |
| IgG- PE | Sigma | P-4685 |

Cells were analyzed at passages 8, 15, and 20, and umbilical cord tissue-derived cells from different donors were compared to each other. In addition, cells cultured on gelatin-coated flasks were compared to cells cultured on uncoated flasks.

Umbilical cord tissue-derived cells showed positive expression of CD10, CD13, CD44, CD73, CD 90, PDGFr-alpha and HLA-A, B, C, indicated by the increased values of fluorescence relative to the IgG control. These cells were negative for detectable expression of CD31, CD34, CD45, CD117, CD141, and HLA-DR, DP, DQ, indicated by fluorescence values comparable to the IgG control. Variations in fluorescence values of positive curves were accounted for. The mean (*i.e.*, CD13) and range (*i.e.*, CD90) of the positive curves showed some variation, but the curves appeared normal, confirming a homogenous population. Both curves individually exhibited values greater than the IgG control.

Cells at passage 8, 15, and 20 all expressed CD10, CD13, CD44, CD73, CD 90, PDGFr-alpha and HLA-A, B, C, indicated by increased fluorescence relative to the IgG control. These cells were negative for CD31, CD34, CD45, CD117, CD141, and HLA-DR, DP, DQ, indicated by fluorescence values consistent with the IgG control.

Isolates from separate donors each showed positive expression of CD10, CD13, CD44, CD73, CD 90, PDGFr-alpha and HLA-A, B, C, reflected in the increased values of fluorescence relative to the IgG control. These cells were negative for expression of CD31, CD34, CD45, CD117, CD141, and HLA-DR, DP, DQ with fluorescence values consistent with the IgG control.

Cells expanded on gelatin and uncoated flasks all were positive for expression of CD10, CD13, CD44, CD73, CD 90, PDGFr-alpha and HLA-A, B, C, with increased values of fluorescence relative to the IgG control. These cells were negative for expression of CD31, CD34, CD45, CD117, CD141, and HLA-DR, DP, DQ, with fluorescence values consistent with the IgG control.

Thus, umbilical cord tissue-derived cells are positive for CD10, CD13, CD44, CD73, CD90, PDGFr-alpha, HLA-A,B,C and negative for CD31, CD34, CD45, CD117, CD141 and HLA-DR, DP, DQ. This identity was consistent between variations in variables including the donor, passage, and culture vessel surface coating. Some variation in individual fluorescence value histogram curve means and ranges was observed, but all positive curves under all conditions tested were normal and expressed fluorescence values greater than the IgG control, thereby confirming that the cells comprise a homogenous population that has positive expression of the markers.

## EXAMPLE 3

### Immunohistochemical Characterization of Cell Phenotypes

Human umbilical cord tissue was harvested and immersion-fixed in 4% (w/v) paraformaldehyde overnight at 4°C. Immunohistochemistry was performed using antibodies directed against the following epitopes: vimentin (1:500; Sigma, St. Louis, MO), desmin (1:150, raised against rabbit; Sigma; or 1:300, raised against mouse; Chemicon, Temecula, CA), alpha-smooth muscle actin (SMA; 1:400; Sigma), cytokeratin 18 (CK18; 1:400; Sigma), von Willebrand Factor (vWF; 1:200; Sigma), and CD34 (human CD34 Class III; 1:100; DAKOCytomation, Carpinteria, CA). In addition, the following markers were tested: anti-human GROalpha - PE (1:100; Becton Dickinson, Franklin Lakes, NJ), anti-human GCP-2 (1:100; Santa Cruz Biotech, Santa Cruz, CA), anti-human oxidized LDL receptor 1 (ox-LDL R1; 1:100; Santa Cruz Biotech), and anti-human NOGO-A (1:100; Santa Cruz Biotech). Fixed specimens were trimmed with a scalpel and placed within OCT embedding compound (Tissue-Tek OCT; Sakura, Torrance, CA) on a dry ice bath containing ethanol. Frozen blocks were then sectioned (10 $\mu$m thick) using a standard cryostat (Leica Microsystems) and mounted onto glass slides for staining.

Immunohistochemistry was performed similar to previous studies (Messina et al., Exper. Neurol., 2003; 184:816-29). In brief, tissue sections were washed with phosphate-buffered saline (PBS) and exposed to a protein blocking solution containing PBS, 4% (v/v) goat serum (Chemicon, Temecula, CA), and 0.3% (v/v) Triton (Triton X-100; Sigma) for 1 hour to access intracellular antigens. In instances where the epitope of interest would be located on the cell surface (CD34, ox-LDL R1), Triton was omitted in all steps of the procedure in order to prevent epitope loss. Furthermore, in instances where the primary antibody was raised against goat (GCP-2, ox-LDL R1, NOGO-A), 3% (v/v) donkey serum was used in place of goat serum throughout the procedure. Primary antibodies, diluted in blocking solution, were then applied to the sections for a period of 4 hours at room temperature. Primary antibody solutions were removed, and cultures washed with PBS prior to application of secondary antibody solutions (1 hour at room temperature) containing block along with goat anti-mouse IgG - Texas Red (1:250; Molecular Probes, Eugene, OR) and/or goat anti-rabbit IgG - Alexa 488 (1:250; Molecular Probes) or donkey anti-goat IgG - FITC (1:150; Santa Cruz Biotech). Cultures were washed, and 10 micromolar DAPI (Molecular Probes) was applied for 10 minutes to visualize cell nuclei.

Fluorescence was visualized using the appropriate fluorescence filter on an Olympus inverted epi-fluorescent microscope (Olympus, Melville, NY). Positive staining was represented by fluorescence signal above control staining. Representative images were captured using a digital color video camera and IMAGE-PRO® software (Media Cybernetics, Carlsbad, CA). For triple-stained samples, each image was taken using only one emission filter at a time.

Vimentin, desmin, SMA, CK18, vWF, and CD34 markers were expressed in a subset of the cells found within umbilical cord. In particular, vWF and CD34 expression were restricted to blood vessels contained within the cord. CD34$^+$ cells were on the innermost layer (lumen side). Vimentin expression was found throughout the matrix and blood vessels of the cord. SMA was limited to the matrix and outer walls of the artery & vein, but not contained with the vessels themselves. CK18 and desmin were observed within the vessels only, desmin being restricted to the middle and outer layers. The expression of GROalpha, GCP-2, ox-LDL R1, and NOGO-A were not observed within umbilical cord tissue.

## EXAMPLE 4

### Oligonucleotide Array Analysis

Affymetrix GeneChip® arrays were used to compare gene expression profiles of umbilical cord tissue-derived cells with fibroblasts, human mesenchymal stem cells, and another cell line derived from human bone marrow. This analysis provided a characterization of the postpartum-derived cells and identified unique molecular markers for these cells.

Human umbilical cords were obtained from National Disease Research Interchange (NDRI, Philadelphia, PA) from normal full term deliveries with patient consent. The tissues were received and cells were isolated as described above. Cells were cultured in Growth Medium (using DMEM-LG) on gelatin-coated tissue culture plastic flasks. The cultures were incubated at 37° C with 5 % $CO_2$.

Human dermal fibroblasts were purchased from Cambrex Incorporated (Walkersville, MD; Lot number 9F0844) and ATCC CRL-1501 (CCD39SK). Both lines were cultured in DMEM/F12 medium (Invitrogen, Carlsbad, CA) with 10% (v/v) fetal bovine serum (Hyclone) and penicillin/streptomycin (Invitrogen). The cells were grown on standard tissue-treated plastic.

Human mesenchymal stem cells (hMSC) were purchased from Cambrex Incorporated (Walkersville, MD; Lot numbers 2F1655, 2F1656 and 2F1657) and cultured according to the manufacturer's specifications in MSCGM Media (Cambrex). The cells were grown on standard tissue cultured plastic at 37 °C with 5 % $CO_2$.

Human iliac crest bone marrow was received from NDRI with patient consent. The marrow was processed according to the method outlined by Ho, et al. (WO 2003/025149). The marrow was mixed with lysis buffer (155 mM $NH_4Cl$, 10 mM $KHCO_3$, and 0.1 mM EDTA, pH 7.2) at a ratio of 1 part bone marrow to 20 parts lysis buffer. The cell suspension was vortexed, incubated for 2 minutes at ambient temperature, and centrifuged for 10 minutes at 500 x g. The supernatant was discarded and the cell pellet was resuspended in Minimal Essential Medium-alpha (Invitrogen) supplemented with 10 % (v/v) fetal bovine serum and 4 mM glutamine. The cells were centrifuged again and the cell pellet was resuspended in fresh medium. The viable mononuclear cells were counted using trypan-blue exclusion (Sigma, St. Louis, MO). The mononuclear cells were seeded in tissue-cultured plastic flasks at 5 x 104 cells/cm$^2$. The cells were incubated at 37 °C with 5% $CO_2$ at either standard atmospheric $O_2$ or at 5% $O_2$. Cells were cultured for 5 days without a media change. Media and non-adherent cells were removed after 5 days of culture. The adherent cells were maintained in culture.

Actively growing cultures of cells were removed from the flasks with a cell scraper in cold PBS. The cells were centrifuged for 5 minutes at 300 x g. The supernatant was removed and the cells were resuspended in fresh PBS and centrifuged again. The supernatant was removed and the cell pellet was immediately frozen and stored at -80° C. Cellular mRNA was extracted and transcribed into cDNA, which was then transcribed into cRNA and biotin-labeled. The biotin-labeled cRNA was hybridized with HG-U133A GeneChip oligonucleotide array (Affymetrix, Santa Clara CA). The hybridization and data collection was performed according to the manufacturer's specifications. Analyses were performed using "Significance Analysis of Microarrays" (SAM) version 1.21 computer software (Stanford University; Tusher et al., Proc. Natl. Acad. Sci. USA, 2002; 98:5116-21).

Fourteen different populations of cells were analyzed. The cells along with passage information, culture substrate, and culture media are listed in Table 1.

| Table 1. Cells analyzed by the microarray study. Cell lines are listed by identification code along with passage at time of analysis, cell growth substrate and growth medium. | | | |
|---|---|---|---|
| **Cell Population** | **Passage** | **Substrate** | **Medium** |
| Umbilical cord (022803) | 2 | Gelatin | DMEM, 15% FBS, 2-ME |
| Umbilical cord (042103) | 3 | Gelatin | DMEM, 15% FBS, 2-ME |
| Umbilical cord (071003) | 4 | Gelatin | DMEM, 15% FBS, 2-ME |
| ICBM (070203) (5% $O_2$) | 3 | Plastic | MEM, 10% FBS |
| ICBM (062703) (std. $O_2$) | 5 | Plastic | MEM, 10% FBS |
| ICBM (062703) (5% $O_2$) | 5 | Plastic | MEM, 10% FBS |
| hMSC (Lot 2F1655) | 3 | Plastic | MSCGM |
| hMSC (Lot 2F1656) | 3 | Plastic | MSCGM |
| hMSC (Lot 2F1657) | 3 | Plastic | MSCGM |
| hFibroblast (9F0844) | 9 | Plastic | DMEM-F12, 10% FBS |
| hFibroblast (CCD39SK) | 4 | Plastic | DMEM-F12, 10% FBS |

The data were evaluated by a Principle Component Analysis, analyzing the 290 genes that were differentially expressed in the cells. This analysis allows for a relative comparison for the similarities between the populations. Table 2 shows the Euclidean distances that were calculated for the comparison of the cell pairs. The Euclidean distances were based on the comparison of the cells based on the 290 genes that were differentially expressed among the cell types. The Euclidean distance is inversely proportional to similarity between the expression of the 290 genes (*i.e.*, the greater the distance, the less similarity exists)

| Table 2. The Euclidean Distances for the Cell Pairs. | |
|---|---|
| **Cell Pair** | **Euclidian Distance** |
| ICBM-hMSC | 24.71 |
| Placenta-umbilical | 25.52 |
| ICBM-Fibroblast | 36.44 |
| ICBM-placenta | 37.09 |
| Fibroblast-MSC | 39.63 |
| ICBM-Umbilical | 40.15 |
| Fibroblast-Umbilical | 41.59 |
| MSC-Placenta | 42.84 |
| MSC-Umbilical | 46.86 |
| ICBM-placenta | 48.41 |

Tables 3 and 4 below show the expression of genes increased in umbilical cord tissue-derived cells (Table 3), and reduced in umbilical cord tissue-derived cells (Table 4). The column entitled "Probe Set ID" refers to the manufacturer's identification code for the sets of several oligonucleotide probes located on a particular site on the chip, which hybridize to the named gene (column "Gene Name"), comprising a sequence that can be found within the NCBI (GenBank) database at the specified accession number (column "NCBI Accession Number").

**Table 3. Genes shown to have specifically increased expression in the umbilical cord tissue-derived cells as compared to other cell lines assayed.**

| Genes Increased in Umbilical cord tissue-Derived Cells | | |
|---|---|---|
| **Probe Set ID** | **Gene Name** | **NCBI Accession Number** |
| 202859_x_at | interleukin 8 | NM_000584 |
| 211506_s_at | interleukin 8 | AF043337 |
| 210222_s_at | reticulon 1 | BC000314 |
| 204470_at | chemokine (C-X-C motif) ligand 1 (melanoma growth stimulating activity | NM_001511 |
| 206336_at | chemokine (C-X-C motif) ligand 6 (granulocyte chemotactic protein 2) | NM_002993 |
| 207850_at | chemokine (C-X-C motif) ligand 3 | NM_002090 |
| 203485_at | reticulon 1 | NM_021136 |
| 202644_s_at | tumor necrosis factor, alpha-induced protein 3 | NM_006290 |

**Table 4. Genes shown to have decreased expression in umbilical cord tissue-derived cells as compared to other cell lines assayed.**

| Genes Decreased in Umbilical cord tissue- and Placenta-Derived Cells | | |
|---|---|---|
| **Probe Set ID** | **Gene name** | **NCBI Accession Number** |
| 210135_s_at | short stature homeobox 2 | AF022654.1 |
| 205824_at | heat shock 27kDa protein 2 | NM_001541.1 |
| 209687_at | chemokine (C-X-C motif) ligand 12 (stromal cell-derived factor 1) | U19495.1 |
| 203666_at | chemokine (C-X-C motif) ligand 12 (stromal cell-derived factor 1) | NM_000609.1 |
| 212670_at | elastin (supravalvular aortic stenosis, Williams-Beuren syndrome) | AA479278 |
| 213381_at | *Homo sapiens* mRNA; cDNA DKFZp586M2022 (from clone DKFZp586M2022) | N91149 |
| 206201_s_at | mesenchyme homeo box 2 (growth arrest-specific homeo box) | NM_005924.1 |
| 205817_at | sine oculis homeobox homolog 1 (*Drosophila*) | NM_005982.1 |
| 209283_at | crystallin, alpha B | AF007162.1 |
| 212793_at | dishevelled associated activator of morphogenesis 2 | BF513244 |
| 213488_at | DKFZP586B2420 protein | AL050143.1 |
| 209763_at | similar to neuralin 1 | AL049176 |
| 205200_at | tetranectin (plasminogen binding protein) | NM_003278.1 |
| 205743_at | src homology three (SH3) and cysteine rich domain | NM_003149.1 |
| 200921_s_at | B-cell translocation gene 1, anti-proliferative | NM_001731.1 |
| 206932_at | cholesterol 25-hydroxylase | NM_003956.1 |
| 204198_s_at | runt-related transcription factor 3 | AA541630 |
| 219747_at | hypothetical protein FLJ23191 | NM_024574.1 |

(continued)

| Genes Decreased in Umbilical cord tissue- and Placenta-Derived Cells | | |
|---|---|---|
| **Probe Set ID** | **Gene name** | **NCBI Accession Number** |
| 204773_at | interleukin 11 receptor, alpha | NM_004512.1 |
| 202465_at | procollagen C-endopeptidase enhancer | NM_002593.2 |
| 203706_s_at | frizzled homolog 7 (*Drosophila*) | NM_003507.1 |
| 212736_at | hypothetical gene BC008967 | BE299456 |
| 214587_at | collagen, type VIII, alpha 1 | BE877796 |
| 201645_at | tenascin C (hexabrachion) | NM_002160.1 |
| 210239_at | iroquois homeobox protein 5 | U90304.1 |
| 203903_s_at | Hephaestin | NM_014799.1 |
| 205816_at | integrin, beta 8 | NM_002214.1 |
| 203069_at | synaptic vesicle glycoprotein 2 | NM_014849.1 |
| 213909_at | *Homo sapiens* cDNA FLJ12280 fis, clone MAMMA1001744 | AU147799 |
| 206315_at | cytokine receptor-like factor 1 | NM_004750.1 |
| 204401_at | potassium intermediate/small conductance calcium-activated channel, subfamily N, member 4 | NM_002250.1 |
| 216331_at | integrin, alpha 7 | AK022548.1 |
| 209663_s_at | integrin, alpha 7 | AF072132.1 |
| 213125_at | DKFZP586L151 protein | AW007573 |
| 202133_at | transcriptional co-activator with PDZ-binding motif (TAZ) | AA081084 |
| 206511_s_at | sine oculis homeobox homolog 2 (*Drosophila*) | NM_016932.1 |
| 213435_at | KIAA1034 protein | AB028957.1 |
| 206115_at | early growth response 3 | NM_004430.1 |
| 213707_s_at | distal-less homeo box 5 | NM_005221.3 |
| 218181_s_at | hypothetical protein FLJ20373 | NM_017792.1 |
| 209160_at | aldo-keto reductase family 1, member C3 (3-alpha hydroxysteroid dehydrogenase, type II) | AB018580.1 |
| 213905_x_at | Biglycan | AA845258 |
| 201261_x_at | Biglycan | BC002416.1 |
| 202132_at | transcriptional co-activator with PDZ-binding motif (TAZ) | AA081084 |
| 214701_s_at | fibronectin 1 | AJ276395.1 |
| 213791_at | Proenkephalin | NM_006211.1 |
| 205422_s_at | integrin, beta-like 1 (with EGF-like repeat domains) | NM_004791.1 |
| 214927_at | *Homo sapiens* mRNA full length insert cDNA clone EUROIMAGE 1968422 | AL359052.1 |
| 206070_s_at | EphA3 | AF213459.1 |
| 212805_at | KIAA0367 protein | AB002365.1 |

(continued)

| Genes Decreased in Umbilical cord tissue- and Placenta-Derived Cells | | |
|---|---|---|
| Probe Set ID | Gene name | NCBI Accession Number |
| 219789_at | natriuretic peptide receptor C/guanylate cyclase C | AI628360 |
| | (atrionatriuretic peptide receptor C) | |
| 219054_at | hypothetical protein FLJ14054 | NM_024563.1 |
| 213429_at | *Homo sapiens* mRNA; cDNA DKFZp564B222 (from clone DKFZp564B222) | AW025579 |
| 204929_s_at | vesicle-associated membrane protein 5 (myobrevin) | NM 006634.1 |
| 201843_s_at | EGF-containing fibulin-like extracellular matrix protein 1 | NM_004105.2 |
| 221478_at | BCL2/adenovirus E1B 19kDa interacting protein 3-like | AL132665.1 |
| 201792_at | AE binding protein 1 | NM_001129.2 |
| 204570_at | cytochrome c oxidase subunit VIIa polypeptide 1 (muscle) | NM_001864.1 |
| 201621_at | neuroblastoma, suppression of tumorigenicity 1 | NM_005380.1 |
| 202718_at | insulin-like growth factor binding protein 2, 36kDa | NM_000597.1 |

Tables 5, 6, and 7 show the expression of genes increased in human fibroblasts (Table 5), ICBM cells (Table 6), and MSCs (Table 7).

| Table 5. Genes that were shown to have increased expression in fibroblasts as compared to the other cell lines assayed. |
|---|
| dual specificity phosphatase 2 |
| KIAA0527 protein |
| Homo sapiens cDNA: FLJ23224 fis, clone ADSU02206 |
| dynein, cytoplasmic, intermediate polypeptide 1 |
| ankyrin 3, node of Ranvier (ankyrin G) |
| inhibin, beta A (activin A, activin AB alpha polypeptide) |
| ectonucleotide pyrophosphatase/phosphodiesterase 4 (putative function) |
| KIAA1053 protein |
| microtubule-associated protein 1A |
| zinc finger protein 41 |
| HSPC019 protein |
| *Homo sapiens* cDNA: FLJ23564 fis, clone LNG10773 |
| *Homo sapiens* mRNA; cDNA DKFZp564A072 (from clone DKFZp564A072) |
| LIM protein (similar to rat protein kinase C-binding enigma) |
| inhibitor of kappa light polypeptide gene enhancer in B-cells, kinase complex-associated protein |
| hypothetical protein FLJ22004 |
| Human (clone CTG-A4) mRNA sequence |
| ESTs, Moderately similar to cytokine receptor-like factor 2; |
| cytokine receptor CRL2 precursor [Homo sapiens] |
| transforming growth factor, beta 2 |

(continued)

| Table 5. Genes that were shown to have increased expression in fibroblasts as compared to the other cell lines assayed. |
| --- |
| hypothetical protein MGC29643 |
| antigen identified by monoclonal antibody MRC OX-2 |
| putative X-linked retinopathy protein |

| Table 6. Genes that were shown to have increased expression in the ICBM-derived cells as compared to the other cell lines assayed. |
| --- |
| •cardiac ankyrin repeat protein |
| •MHC class I region ORF |
| •integrin, alpha 10 |
| •hypothetical protein FLJ22362 |
| •UDP-N-acetyl-alpha-D-galactosamine:polypeptide N-acetylgalactosaminyltransferase 3 (GalNAc-T3) |
| •interferon-induced protein 44 |
| •SRY (sex determining region Y)-box 9 (campomelic dysplasia, autosomal sex-reversal) |
| •keratin associated protein 1-1 |
| •hippocalcin-like 1 |
| •jagged 1 (Alagille syndrome) |
| •proteoglycan 1, secretory granule |

| Table 7. Genes that were shown to have increased expression in the MSC cells as compared to the other cell lines assayed. |
| --- |
| •interleukin 26 |
| •maltase-glucoamylase (alpha-glucosidase) |
| •nuclear receptor subfamily 4, group A, member 2 |
| •v-fos FBJ murine osteosarcoma viral oncogene homolog |
| •hypothetical protein DC42 |
| •nuclear receptor subfamily 4, group A, member 2 |
| •FBJ murine osteosarcoma viral oncogene homolog B |
| •WNT1 inducible signaling pathway protein 1 |
| •MCF.2 cell line derived transforming sequence |
| •potassium channel, subfamily K, member 15 |
| •cartilage paired-class homeoprotein 1 |
| •Homo sapiens cDNA FLJ12232 fis, clone MAMMA1001206 |
| •Homo sapiens cDNA FLJ34668 fis, clone LIVER2000775 |
| •jun B proto-oncogene |
| •B-cell CLL/lymphoma 6 (zinc finger protein 51) |
| •zinc finger protein 36, C3H type, homolog (mouse) |

The foregoing analysis included cells derived from three different umbilical cords and two different lines of dermal fibroblasts, three lines of mesenchymal stem cells, and three lines of iliac crest bone marrow cells. The mRNA that was expressed by these cells was analyzed using an oligonucleotide array that contained probes for 22,000 genes. Results showed that 290 genes are differentially expressed in these five different cell types. These genes include seven genes specifically increased in the umbilical cord tissue-derived cells. Fifty-four genes were found to have specifically lower expression levels in umbilical cord tissue-derived cells, as compared with the other cell types. The expression of selected genes has been confirmed by PCR. These results demonstrate that umbilical cord tissue-derived cells have a distinct gene expression profile, for example, as compared to bone marrow-derived cells and fibroblasts.

## EXAMPLE 5

## Cell Markers in Umbilical Cord Tissue-Derived Cells

As demonstrated above, "signature" genes were identified for postpartum-derived cells: oxidized LDL receptor 1, interleukin-8, renin, reticulon, chemokine receptor ligand 3 (CXC ligand 3), and granulocyte chemotactic protein 2 (GCP-2). These "signature" genes were expressed at relatively high levels in postpartum-derived cells.

The procedures described in this example were conducted to verify the microarray data and find concordance/discordance between gene and protein expression, as well as to establish a series of reliable assay for detection of unique identifiers for umbilical cord tissue-derived cells.

Umbilical cord tissue-derived cells (four isolates), and Normal Human Dermal Fibroblasts (NHDF; neonatal and adult) were grown in Growth Medium with penicillin/streptomycin in a gelatin-coated T75 flask. Mesenchymal Stem Cells (MSCs) were grown in Mesenchymal Stem Cell Growth Medium Bullet kit (MSCGM; Cambrex, Walkersville, MD).

For the IL-8 protocol, cells were thawed from liquid nitrogen and plated in gelatin-coated flasks at 5,000 cells/cm$^2$, grown for 48 hours in Growth Medium and then grown for further 8 hours in 10 milliliters of serum starvation medium (DMEM-low glucose (Gibco, Carlsbad, CA), penicillin/streptomycin (Gibco, Carlsbad, CA) and 0.1% (w/v) Bovine Serum Albumin (BSA; Sigma, St. Louis, MO)). After this treatment, RNA was extracted and the supernatants were centrifuged at 150 x g for 5 minutes to remove cellular debris. Supernatants were then frozen at -80°C for ELISA analysis.

Postpartum cells derived from the umbilical cord, as well as human fibroblasts derived from human neonatal foreskin were cultured in Growth Medium in gelatin-coated T75 flasks. Cells were frozen at passage 11 in liquid nitrogen. Cells were thawed and transferred to 15-milliliter centrifuge tubes. After centrifugation at 150 x g for 5 minutes, the supernatant was discarded. Cells were resuspended in 4 milliliters culture medium and counted. Cells were grown in a 75 cm$^2$ flask containing 15 milliliters of Growth Medium at 375,000 cell/flask for 24 hours. The medium was changed to a serum starvation medium for 8 hours. Serum starvation medium was collected at the end of incubation, centrifuged at 14,000 x g for 5 minutes (and stored at -20 °C).

To estimate the number of cells in each flask, 2 milliliters of trypsin/EDTA (Gibco, Carlsbad, CA) was added each flask. After cells detached from the flask, trypsin activity was neutralized with 8 milliliters of Growth Medium. Cells were transferred to a 15 milliliters centrifuge tube and centrifuged at 150 x g for 5 minutes. Supernatant was removed and 1 milliliter Growth Medium was added to each tube to resuspend the cells. Cell number was estimated using a hemocytometer.

The amount of IL-8 secreted by the cells into serum starvation medium was analyzed using ELISA assays (R&D Systems, Minneapolis, MN). All assays were tested according to the instructions provided by the manufacturer.

RNA was extracted from confluent umbilical cord tissue-derived cells and fibroblasts or for IL-8 expression from cells treated as described above. Cells were lysed with 350 microliters buffer RLT containing beta-mercaptoethanol (Sigma, St. Louis, MO) according to the manufacturer's instructions (RNeasy® Mini Kit; Qiagen, Valencia, CA). RNA was extracted according to the manufacturer's instructions (RNeasy® Mini Kit; Qiagen, Valencia, CA) and subjected to DNase treatment (2.7 U/sample) (Sigma St. Louis, MO). RNA was eluted with 50 microliters DEPC-treated water and stored at -80°C.

RNA was also extracted from human umbilical cord tissue. Tissue (30 milligram) was suspended in 700 microliters of buffer RLT containing 2-mercaptoethanol. Samples were mechanically homogenized and the RNA extraction proceeded according to manufacturer's specification. RNA was extracted with 50 microliters of DEPC-treated water and stored at -80°C. RNA was reversed transcribed using random hexamers with the TaqMan reverse transcription reagents (Applied Biosystems, Foster City, CA) at 25°C for 10 minutes, 37°C for 60 minutes, and 95°C for 10 minutes. Samples were stored at -20°C.

Genes identified by cDNA microarray as uniquely regulated in postpartum cells (signature genes - including oxidized LDL receptor, interleukin-8, renin and reticulon), were further investigated using real-time and conventional PCR.

PCR was performed on cDNA samples using Assays-on-Demand™ gene expression products: oxidized LDL receptor (Hs00234028); renin (Hs00166915); reticulon (Hs00382515); CXC ligand 3 (Hs00171061); GCP-2 (Hs00605742); IL-8 (Hs00174103); and GAPDH (Applied Biosystems, Foster City, CA) were mixed with cDNA and TaqMan Universal PCR master mix according to the manufacturer's instructions (Applied Biosystems, Foster City, CA) using a 7000 sequence detection system with ABI Prism 7000 SDS software (Applied Biosystems, Foster City, CA). Thermal cycle conditions were initially 50°C for 2 min and 95°C for 10 min, followed by 40 cycles of 95°C for 15 sec and 60°C for 1 min. PCR data was analyzed according to manufacturer's specifications (User Bulletin #2 from Applied Biosystems for ABI Prism 7700 Sequence Detection System).

Conventional PCR was performed using an ABI PRISM 7700 (Perkin Elmer Applied Biosystems, Boston, Massachusetts, USA) to confirm the results from real-time PCR. PCR was performed using 2 microliters of cDNA solution, 1 × AmpliTaq Gold universal mix PCR reaction buffer (Applied Biosystems, Foster City, CA) and initial denaturation at 94°C for 5 minutes. Amplification was optimized for each primer set. For IL-8, CXC ligand 3, and reticulon (94°C for 15 seconds, 55°C for 15 seconds and 72°C for 30 seconds for 30 cycles); for renin (94°C for 15 seconds, 53°C for 15 seconds and 72°C for 30 seconds for 38 cycles); for oxidized LDL receptor and GAPDH (94°C for 15 seconds, 55°C for 15 seconds and 72°C for 30 seconds for 33 cycles). Primers used for amplification are listed in Table 8. Primer concentration in the final PCR reaction was 1 micromolar except for GAPDH, which was 0.5 micromolar. GAPDH primers were the same as real-time PCR, except that the manufacturer's TaqMan probe was not added to the final PCR reaction. Samples were run on 2% (w/v) agarose gel and stained with ethidium bromide (Sigma, St. Louis, MO). Images were captured using a 667 Universal Twinpack film (VWR International, South Plainfield, NJ) using a focal-length Polaroid camera (VWR International, South Plainfield, NJ).

**Table 8: Primers used**

| Primer Name | Primers |
|---|---|
| Oxidized LDL receptor | S: 5'-GAGAAATCCAAAGAGCAAATGG-3'(SEQ ID NO:1) |
| | A: 5'-AGAATGGAAAACTGGAATAGG-3'(SEQ ID NO:2) |
| Renin | S: 5'-TCTTCGATGCTTCGGATTCC-3'(SEQ ID NO:3) |
| | A: 5'-GAATTCTCGGAATCTCTGTTG-3' (SEQ ID NO:4) |
| Reticulon | S: 5'-TTACAAGCAGTGCAGAAAACC-3'(SEQ ID NO:5) |
| | A: 5'-AGTAAACATTGAAACCACAGCC-3' (SEQ ID NO:6) |
| Interleukin-8 | S: 5'-TCTGCAGCTCTGTGTGAAGG-3'(SEQ ID NO:7) |
| | A: 5'-CTTCAAAAACTTCTCCACAACC-3'(SEQ ID NO:8) |
| Chemokine (CXC) ligand 3 | S: 5'-CCCACGCCACGCTCTCC-3' (SEQ ID NO: 9) |
| | A: 5'-TCCTGTCAGTTGGTGCTCC-3'(SEQ ID NO:10) |

Cells were fixed with cold 4% (w/v) paraformaldehyde (Sigma-Aldrich, St. Louis, MO) for 10 minutes at room temperature. One isolate at passage 0 (P0) (directly after isolation) and two isolates at passage 11 (P11), and fibroblasts (P11) were used. Immunocytochemistry was performed using antibodies directed against the following epitopes: vimentin (1:500, Sigma, St. Louis, MO), desmin (1:150; Sigma - raised against rabbit; or 1:300; Chemicon, Temecula, CA-raised against mouse,), alpha-smooth muscle actin (SMA; 1:400; Sigma), cytokeratin 18 (CK18; 1:400; Sigma), von Willebrand Factor (vWF; 1:200; Sigma), and CD34 (human CD34 Class III; 1:100; DAKOCytomation, Carpinteria, CA). In addition, the following markers were tested on passage 11 postpartum cells: anti-human GRO alpha - PE (1:100; Becton Dickinson, Franklin Lakes, NJ), anti-human GCP-2 (1:100; Santa Cruz Biotech, Santa Cruz, CA), anti-human oxidized LDL receptor 1 (ox-LDL R1; 1:100; Santa Cruz Biotech), and anti-human NOGA-A (1:100; Santa Cruz, Biotech).

Cultures were washed with phosphate-buffered saline (PBS) and exposed to a protein blocking solution containing PBS, 4% (v/v) goat serum (Chemicon, Temecula, CA), and 0.3% (v/v) Triton (Triton X-100; Sigma, St. Louis, MO) for 30 minutes to access intracellular antigens. Where the epitope of interest was located on the cell surface (CD34, ox-LDL R1), Triton X-100 was omitted in all steps of the procedure in order to prevent epitope loss. Furthermore, in instances where the primary antibody was raised against goat (GCP-2, ox-LDL R1, NOGO-A), 3% (v/v) donkey serum was used in place of goat serum throughout. Primary antibodies, diluted in blocking solution, were then applied to the cultures for a period of 1 hour at room temperature. The primary antibody solutions were removed and the cultures were washed with PBS prior to application of secondary antibody solutions (1 hour at room temperature) containing block along with goat anti-mouse IgG-Texas Red (1:250; Molecular Probes, Eugene, OR) and/or goat anti-rabbit IgG - Alexa 488 (1:250; Molecular Probes) or donkey anti-goat IgG - FITC (1:150, Santa Cruz Biotech). Cultures were then washed and 10 micromolar DAPI (Molecular Probes) applied for 10 minutes to visualize cell nuclei.

Following immunostaining, fluorescence was visualized using an appropriate fluorescence filter on an Olympus inverted epi-fluorescent microscope (Olympus, Melville, NY). In all cases, positive staining represented fluorescence signal above control staining where the entire procedure outlined above was followed with the exception of application of a primary antibody solution. Representative images were captured using a digital color video camera and IMAGE-PRO® software (Media Cybernetics, Carlsbad, CA). For triple-stained samples, each image was taken using only one emission filter at a time. Layered montages were then prepared using Adobe Photoshop software (Adobe, San Jose, CA).

Adherent cells in flasks were washed in phosphate buffered saline (PBS) (Gibco, Carlsbad, CA) and detached with Trypsin/EDTA (Gibco, Carlsbad, CA). Cells were harvested, centrifuged, and re-suspended 3% (v/v) FBS in PBS at a cell concentration of $1 \times 10^7$ per milliliter. One hundred microliter aliquots were delivered to conical tubes. Cells stained for intracellular antigens were permeabilized with Perm/Wash buffer (BD Pharmingen, San Diego, CA). Antibody was added to aliquots as per manufactures specifications and the cells were incubated for in the dark for 30 minutes at 4°C. After incubation, cells were washed with PBS and centrifuged to remove excess antibody. Cells requiring a secondary antibody were resuspended in 100 microliters of 3% FBS. Secondary antibody was added as per manufactures specification and the cells were incubated in the dark for 30 minutes at 4°C. After incubation, cells were washed with PBS and centrifuged to remove excess secondary antibody. Washed cells were resuspended in 0.5 milliliters PBS and analyzed by flow cytometry. The following antibodies were used: oxidized LDL receptor 1. (sc-5813; Santa Cruz, Biotech), GROa (555042; BD Pharmingen, Bedford, MA), Mouse IgG1 kappa, (P-4685 and M-5284; Sigma), Donkey against Goat IgG (sc-3743; Santa Cruz, Biotech.). Flow cytometry analysis was performed with FACScalibur (Becton Dickinson San Jose, CA).

The data obtained from real-time PCR were analyzed by the $\Delta\Delta$CT method and expressed on a logarithmic scale. Levels of reticulon and oxidized LDL receptor expression were higher in umbilical cord tissue-derived cells as compared to other cells. No significant difference in the expression levels of CXC ligand 3 and GCP-2 were found between postpartum-derived cells and controls. The results of real-time PCR were confirmed by conventional PCR. Sequencing of PCR products further validated these observations. No significant difference in the expression level of CXC ligand 3 was found between postpartum-derived cells and controls using conventional PCR CXC ligand 3 primers listed above.

The production of the cytokine IL-8 in postpartum cells was elevated in both Growth Medium-cultured and serum-starved postpartum-derived cells. All real-time PCR data was validated with conventional PCR and by sequencing PCR products.

When supernatants of cells grown in serum-free medium were examined for the presence of IL-8, the highest amounts were detected in media derived from umbilical cells and some isolates of placenta cells (Table 9). No IL-8 was detected in medium derived from human dermal fibroblasts.

| Table 9. IL-8 protein amount measured by ELISA | |
|---|---|
| **Cell type** | **IL-8** |
| hFibro | ND |
| Umb Isolate 1 | 2058.42 ± 144.67 |
| Umb Isolate 2 | 2368.86 ± 22.73 |
| Values picograms/million cells, n=2, sem; ND= Not Detected | |

Cells derived from the human umbilical cord tissue at passage 0 were probed for the production of selected proteins by immunocytochemical analysis. Immediately after isolation (passage 0), cells were fixed with 4% paraformaldehyde and exposed to antibodies for six proteins: von Willebrand Factor, CD34, cytokeratin 18, desmin, alpha-smooth muscle actin, and vimentin. Umbilical cord tissue-derived cells were positive for alpha-smooth muscle actin and vimentin, with the staining pattern consistent through passage 11.

Concordance between gene expression levels measured by microarray and PCR (both real-time and conventional) has been established for four genes: oxidized LDL receptor 1, renin, reticulon, and IL-8. The expression of these genes was differentially regulated at the mRNA level in PPDCs, with IL-8 also differentially regulated at the protein level. Cells derived from the human umbilical cord tissue at passage 0 were probed for the expression of alpha-smooth muscle actin and vimentin, and were positive for both. The staining pattern was preserved through passage 11.

**EXAMPLE 6**

**_In Vitro_ Immunological Evaluation of Postpartum -Derived Cells**

Postpartum-derived cells (PPDCs) were evaluated _in vitro_ for their immunological characteristics in an effort to predict the immunological response, if any, these cells would elicit upon _in vivo_ transplantation. PPDCs were assayed by flow cytometry for the presence of HLA-DR, HLA-DP, HLA-DQ, CD80, CD86, and B7-H2. These proteins are expressed by antigen-presenting cells (APC) and are required for the direct stimulation of naïve CD4[+] T cells (Abbas & Lichtman, Cellular and Molecular Immunology, 5th Ed. (Saunders, Philadelphia, 2003; p. 171)). The cell lines were also analyzed by flow cytometry for the expression of HLA-G (Abbas & Lichtman, 2003, *supra*), CD 178 (Coumans, et al., Journal of Immunological Methods, 1999; 224:185-196), and PD-L2 (Abbas & Lichtman, 2003, *supra;* Brown, et. al., The Journal of Immunology, 2003; 170:1257-1266). The expression of these proteins by cells residing in placental tissues is thought to mediate the immuno-privileged status of placental tissues *in utero.* To predict the extent to which placenta- and umbilical cord tissue-derived cell lines elicit an immune response *in vivo,* the cell lines were tested in a one-way mixed lymphocyte reaction (MLR).

Cells were cultured to confluence in Growth Medium containing penicillin/streptomycin in T75 flasks (Corning, Corning, NY) coated with 2% gelatin (Sigma, St. Louis, MO).

Cells were washed in phosphate buffered saline (PBS) (Gibco, Carlsbad, CA) and detached with Trypsin/EDTA (Gibco, Carlsbad, MO). Cells were harvested, centrifuged, and re-suspended in 3% (v/v) FBS in PBS at a cell concentration of $1x10^7$ per milliliter. Antibody (Table 10) was added to one hundred microliters of cell suspension as per manufacturer's specifications and incubated in the dark for 30 minutes at 4°C. After incubation, cells were washed with PBS and centrifuged to remove unbound antibody. Cells were re-suspended in five hundred microliters of PBS and analyzed by flow cytometry using a FACSCalibur instrument (Becton Dickinson, San Jose, CA).

| Table 10. Antibodies | | |
|---|---|---|
| **Antibody** | **Manufacturer** | **Catalog Number** |
| HLA-DRDPDQ | BD Pharmingen (San Diego, CA) | 555558 |
| CD80 | BD Pharmingen (San Diego, CA) | 557227 |
| CD86 | BD Pharmingen (San Diego, CA) | 555665 |
| B7-H2 | BD Pharmingen (San Diego, CA) | 552502 |
| HLA-G | Abcam (Cambridgeshire, UK) | ab 7904-100 |
| CD 178 | Santa Cruz (San Cruz, CA) | sc-19681 |
| PD-L2 | BD Pharmingen (San Diego, CA) | 557846 |
| Mouse IgG2a | Sigma (St. Louis, MO) | F-6522 |
| Mouse IgG1kappa | Sigma (St. Louis, MO) | P-4685 |

Cryopreserved vials of passage 10 umbilical cord tissue-derived cells labeled as cell line A were sent on dry ice to CTBR (Senneville, Quebec) to conduct a mixed lymphocyte reaction using CTBR SOP No. CAC-031. Peripheral blood mononuclear cells (PBMCs) were collected from multiple male and female volunteer donors. Stimulator (donor) allergenic PBMC, autologous PBMC, and postpartum cell lines were treated with mitomycin C. Autologous and mitomycin C-treated stimulator cells were added to responder (recipient) PBMCs and cultured for 4 days. After incubation, [$^3$H]thymidine was added to each sample and cultured for 18 hours. Following harvest of the cells, radiolabeled DNA was extracted, and [$^3$H]-thymidine incorporation was measured using a scintillation counter.

The stimulation index for the allogeneic donor (SIAD) was calculated as the mean proliferation of the receiver plus mitomycin C-treated allogeneic donor divided by the baseline proliferation of the receiver. The stimulation index of the PPDC was calculated as the mean proliferation of the receiver plus mitomycin C-treated postpartum cell line divided by the baseline proliferation of the receiver.

Six human volunteer blood donors were screened to identify a single allogeneic donor that will exhibit a robust proliferation response in a mixed lymphocyte reaction with the other five blood donors. This donor was selected as the allogeneic positive control donor. The remaining five blood donors were selected as recipients. The allogeneic positive control donor and placenta cell lines were mitomycin C-treated and cultured in a mixed lymphocyte reaction with the five individual allogeneic receivers. Reactions were performed in triplicate using two cell culture plates with three receivers per plate (Table 11). The average stimulation index ranged from 6.5 (plate 1) to 9 (plate 2) and the allogeneic donor positive controls ranged from 42.75 (plate 1) to 70 (plate 2) (Table 12).

**Table 11. Mixed Lymphocyte Reaction Data- Cell Line A (Umbilical cord)**

| Analytical number | Culture System | | Replicates | | | | Mean | SD | CV |
|---|---|---|---|---|---|---|---|---|---|
| | | DPM for Proliferation Assay Plate ID: Plate1 | | | | | | | |
| | | | 1 | 2 | 3 | | | | |
| IM04-2478 | Proliferation baseline of receiver | | 1074 | 406 | 391 | | 623.7 | 390.07 | 62.5 |
| | Control of autostimulation (Mitomycin C treated autologous cells) | | 672 | 510 | 1402 | | 861.3 | 475.19 | 55.2 |
| | MLR allogenic donor IM04-2477 (Mitomycin C treated) | | 43777 | 48391 | 38231 | | 43466.3 | 5087.12 | 11.7 |
| | MLR with cell line (Mitomycin C treated cell type A) | | 2914 | 5622 | 6109 | | 4881.7 | 1721.36 | 35.3 |
| SI (donor) SI (cell line) | | | | | | | 70 8 | | |
| IM04-2479 | Proliferation baseline of receiver | | 530 | 508 | 527 | | 521.7 | 11.93 | 2.3 |
| | Control of autostimulation (Mitomycin C treated autologous cells) | | 701 | 567 | 1111 | | 793.0 | 283.43 | 35.7 |
| | MIR allogenic donor IM04-2477 (Mitomycin C treated) | | 25593 | 24732 | 22707 | | 24344.0 | 1481.61 | 6.1 |
| | MLR with cell line (Mitomycin C treated cell type A) | | 5086 | 3932 | 1497 | | 3505.0 | 1832.21 | 52.3 |
| SI (donor) SI (cell line) | | | | | | | 47 7 | | |
| IM04-2480 | Proliferation baseline of receiver | | 1192 | 854 | 1330 | | 1125.3 | 244.90 | 21.8 |
| | Control of autostimulation (Mitomycin C treated autologous cells) | | 2963 | 993 | 2197 | | 2051.0 | 993.08 | 48.4 |
| | MLR allogenic donor IM04-2477 (Mitomycin C treated) | | 25416 | 29721 | 23757 | | 26298.0 | 3078.27 | 11.7 |
| | MLR with cell line (Mitomycin C treated cell type A) | | 2596 | 5076 | 3426 | | 3699.3 | 1262.39 | 34.1 |
| SI(donor) SI (cell line) | | | | | | | 23 3 | | |
| | Proliferation baseline of receiver | | 695 | 451 | 555 | | 567.0 | 122.44 | 21.6 |

(continued)

| DPM for Proliferation Assay<br>Plate ID: Plate1 | | | | | | | | |
|---|---|---|---|---|---|---|---|---|
| Analytical<br>number | Culture<br>System | | Replicates<br>1 | 2 | 3 | Mean | SD | CV |
| IM04-2481 | Control of autostimulation (Mitomycin C treated autologous cells) | | 738 | 1252 | 464 | 818.0 | 400.04 | 48.9 |
| | MLR allogenic donor IM04-2477 (Mitomycin C treated) | | 13177 | 24885 | 15444 | 17835.3 | 6209.52 | 34.8 |
| | MLR with cell line (Mitomycin C treated cell type A) | | 4495 | 3671 | 4674 | 4280.0 | 534.95 | 12.5 |
| SI (donor)<br>SI (cell line) | | | | | | 31<br>8 | | |
| | | | | | | | | |
| Plate ID: Plate 2 | | | | | | | | |
| Analytical<br>number | Culture<br>System | | Replicates<br>1 | 2 | 3 | Mean | SD | CV |
| IM04-2482 | Proliferation baseline of receiver | | 432 | 533 | 274 | 413.0 | 130.54 | 31.6 |
| | Control of autostimulation (Mitomycin C treated autologous cells) | | 1459 | 633 | 598 | 896.7 | 487.31 | 54.3 |
| | MLR allogenic donor IM04-2477 (Mitomycin C treated) | | 24286 | 30823 | 31346 | 28818.3 | 3933.82 | 13.7 |
| | MLR with cell line (Mitomycin C treated cell type A) | | 2762 | 1502 | 6723 | 3662.3 | 2724.46 | 74.4 |
| SI (donor)<br>SI (cell line) | | | | | | 70<br>9 | | |
| IM04-2477 (allogenic donor) | Proliferation baseline of receiver | | 312 | 419 | 349 | 360.0 | 54.34 | 15.1 |
| | Control of autostimulation (Mitomycin treated autologous cells) | | 567 | 604 | 374 | 515.0 | 123.50 | 24.0 |
| | | | | | | | | |
| Cell line type A | Proliferation baseline of receiver | | 5101 | 3735 | 2973 | 3936.3 | 1078.19 | 27.4 |
| | Control of autostimulation (Mitomycin treated autologous cells) | | 1924 | 4570 | 2153 | 2882.3 | 1466.04 | 50.9 |
| | | | | | | | | |

**Table 12. Average stimulation index of umbilical cord tissue-derived cells and an allogeneic donor in a mixed lymphocyte reaction with five individual allogeneic receivers.**

| Average Stimulation Index | | |
|---|---|---|
| | Recipient | Umbilicus |
| Plate 1 (receivers 1-4) | 42.75 | 6.5 |
| Plate 2 (receiver 5) | 70 | 9 |

Histograms of umbilical cord tissue-derived cells analyzed by flow cytometry show negative expression of HLA-DR, DP, DQ, CD80, CD86, and B7-H2, as noted by fluorescence value consistent with the IgG control, indicating that umbilical cell lines lack the cell surface molecules required to directly stimulate CD4+ T cells. Histograms of umbilical cord tissue-derived cells analyzed by flow cytometry show positive expression of PD-L2, as noted by the increased value of fluorescence relative to the IgG control, and negative expression of CD178 and HLA-G, as noted by fluorescence value consistent with the IgG control.

In the mixed lymphocyte reactions conducted with umbilical cord tissue-derived cell lines the average stimulation index ranged from 6.5 to 9, and that of the allogeneic positive controls ranged from 42.75 to 70. Umbilical cord tissue-derived cell lines were negative for the expression of the stimulating proteins HLA-DR, HLA-DP, HLA-DQ, CD80, CD86, and B7-H2, as measured by flow cytometry. Umbilical cord tissue-derived cell lines were negative for the expression of immuno-modulating proteins HLA-G and CD178 and positive for the expression of PD-L2, as measured by flow cytometry. Allogeneic donor PBMCs contain antigen-presenting cells expressing HLA-DR, DQ, CD8, CD86, and B7-H2, thereby allowing for the stimulation of naïve CD4+ T cells. The absence of antigen-presenting cell surface molecules on placenta- and umbilical cord tissue-derived cells required for the direct stimulation of naive CD4+ T cells and the presence of PD-L2, an immunomodulating protein, may account for the low stimulation index exhibited by these cells in a MLR as compared to allogeneic controls.

## EXAMPLE 7

### Secretion of Trophic Factors by Umbilical Cord Tissue-Derived Cells

The secretion of selected trophic factors from umbilical cord tissue-derived cells was measured. Factors selected for detection included: (1) those known to have angiogenic activity, such as hepatocyte growth factor (HGF) (Rosen et al., Ciba Found. Symp., 1997; 212:215-26), monocyte chemotactic protein 1 (MCP-1) (Salcedo et al., Blood, (2000; 96:34-40), interleukin-8 (IL-8) (Li et al., J. Immunol., 2003; 170:3369-76), keratinocyte growth factor (KGF), basic fibroblast growth factor (bFGF), vascular endothelial growth factor (VEGF) (Hughes et al., Ann. Thorac. Surg., 2004; 77:812-8), matrix metalloproteinase 1 (TIMP1), angiopoietin 2 (ANG2), platelet derived growth factor (PDGF-bb), thrombopoietin (TPO), heparin-binding epidermal growth factor (HB-EGF), stromal-derived factor 1alpha (SDF-1alpha); (2) those known to have neurotrophic/neuroprotective activity, such as brain-derived neurotrophic factor (BDNF) (Cheng et al., Dev. Biol., 2003; 258:319-33), interleukin-6 (IL-6), granulocyte chemotactic protein-2 (GCP-2), transforming growth factor beta2 (TGFbeta2); and (3) those known to have chemokine activity, such as macrophage inflammatory protein 1alpha (MIP1a), macrophage inflammatory protein 1beta (MIP1b), monocyte chemoattractant-1 (MCP-1), Rantes (regulated on activation, normal T cell expressed and secreted), I309, thymus and activation-regulated chemokine (TARC), Eotaxin, macrophage-derived chemokine (MDC), IL-8.

Cells from the umbilical cord as well as human fibroblasts derived from human neonatal foreskin were cultured in Growth Medium with penicillin/streptomycin on gelatin-coated T75 flasks. Cells were cryopreserved at passage 11 and stored in liquid nitrogen. After thawing of the cells, Growth Medium was added to the cells followed by transfer to a 15 milliliter centrifuge tube and centrifugation of the cells at 150 x g for 5 minutes. The supernatant was discarded. The cell pellet was resuspended in 4 milliliters Growth Medium, and cells were counted. Cells were seeded at 375,000 cells/75 cm$^2$ flask containing 15 milliliters of Growth Medium and cultured for 24 hours. The medium was changed to a serum-free medium (DMEM-low glucose (Gibco), 0.1% (w/v) bovine serum albumin (Sigma), penicillin/streptomycin (Gibco)) for 8 hours. Conditioned serum-free medium was collected at the end of incubation by centrifugation at 14,000 x g for 5 minutes and stored at -20°C. To estimate the number of cells in each flask, cells were washed with PBS and detached using 2 milliliters trypsin/EDTA. Trypsin activity was inhibited by addition of 8 milliliters Growth Medium. Cells were centrifuged at 150 x g for 5 minutes. Supernatant was removed, and cells were resuspended in 1 milliliter Growth Medium. Cell number was estimated using a hemocytometer.

Cells were grown at 37°C in 5% carbon dioxide and atmospheric oxygen. Placenta-derived cells (batch 101503) also were grown in 5% oxygen or beta-mercaptoethanol (BME). The amount of MCP-1, IL-6, VEGF, SDF-1alpha, GCP-2, IL-8, and TGF-beta 2 produced by each cell sample was measured by an ELISA assay (R&D Systems, Minneapolis, MN). All assays were performed according to the manufacturer's instructions.

Chemokines (MIP1a, MIP1b, MCP-1, Rantes, I309, TARC, Eotaxin, MDC, IL8), BDNF, and angiogenic factors (HGF, KGF, bFGF, VEGF, TIMP1, ANG2, PDGF-bb, TPO, HBF-EGF,□ were measured using SEARCHLIGHT® Proteome Arrays (Pierce Biotechnology Inc.). The Proteome Arrays are multiplexed sandwich ELISAs for the quantitative measurement of two to 16 proteins per well. The arrays are produced by spotting a 2 x 2, 3 x 3, or 4 x 4 pattern of four to 16 different capture antibodies into each well of a 96-well plate. Following a sandwich ELISA procedure, the entire plate is imaged to capture chemiluminescent signal generated at each spot within each well of the plate. The amount of signal generated in each spot is proportional to the amount of target protein in the original standard or sample.

MCP-1 and IL-6 were secreted by umbilical cord tissue-derived cells and dermal fibroblasts (Table 13). SDF-1alpha was secreted by fibroblasts. GCP-2 and IL-8 were secreted by umbilical cord tissue-derived cells cultured in the presence of BME or 5% $O_2$. GCP-2 also was secreted by human fibroblasts. TGF-beta2 was not detectable by ELISA assay.

| Table 13. ELISA assay results | | | | | | | |
|---|---|---|---|---|---|---|---|
| | MCP-1 | IL-6 | VEGF | SDF-1$\alpha$ | GCP-2 | IL-8 | TGF-beta2 |
| Fibroblast | 17±1 | 61±3 | 29±2 | 19±1 | 21±1 | ND | ND |
| Umbilical cord (022803) | 1150±74 | 4234±289 | ND | ND | 160±11 | 2058±145 | ND |
| Umbilical cord (071003) | 2794±84 | 1356±43 | ND | ND | 2184±98 | 2369±23 | ND |
| values presented are picograms/milliliter/million cells (n=2, sem); ND = Not Detected. | | | | | | | |

TIMP1, TPO, KGF, HGF, FGF, HBEGF, BDNF, MIP1b, MCP1, RANTES, I309, TARC, MDC, and IL-8 were secreted from umbilical cord tissue-derived cells (Tables 14 and 15). No Ang2, VEGF, or PDGF-bb was detected.

| Table 14. SearchLight® Multiplexed ELISA assay results | | | | | | | | | |
|---|---|---|---|---|---|---|---|---|---|
| | TIMP1 | ANG2 | PDGFbb | TPO | KGF | HGF | FGF | VEGF | HBEGF | BDNF |
| Hfb | 19306.3 | ND | ND | 230.5 | 5.0 | ND | ND | 27.9 | 1.3 | ND |
| U1 | 57718.4 | ND | ND | 1240.0 | 5.8 | 559.3 | 148.7 | ND | 9.3 | 165.7 |
| U3 | 21850.0 | ND | ND | 1134.5 | 9.0 | 195.6 | 30.8 | ND | 5.4 | 388.6 |
| hFB = human fibroblasts, U1 = umbilical cord tissue-derived cells (022803), U3 = umbilical cord tissue-derived cells (071003), ND = Not Detected. | | | | | | | | | |

| Table 15. SearchLight® Multiplexed ELISA assay results | | | | | | | | |
|---|---|---|---|---|---|---|---|---|
| | MIP1a | MIP1b | MCP1 | RANTES | 1309 | TARC | Eotaxin | MDC | IL8 |
| hFB | ND | ND | 39.6 | ND | ND | 0.1 | ND | ND | 204.9 |
| P1 | 79.5 | ND | 228.4 | 4.1 | ND | 3.8 | 12.2 | ND | 413.5 |
| U1 | ND | 8.0 | 1694.2 | ND | 22.4 | 37.6 | ND | 18.9 | 51930.1 |
| P3 | ND | ND | 102.7 | ND | ND | 0.4 | ND | ND | 63.8 |
| U3 | ND | 5.2 | 2018.7 | 41.5 | 11.6 | 21.4 | ND | 4.8 | 10515.9 |
| hFB = human fibroblasts, U1 = umbilical cord tissue-derived cells (022803), U3 = umbilical cord tissue-derived cells (071003), ND = Not Detected. | | | | | | | | |

Umbilical cord tissue-derived cells secreted a number of highly beneficial trophic factors. Some of these trophic factors, such as TIMP1, a catabolic inhibitor, plays a critical role in prevention of extracellular matrix degradation by matrix metaloproteases. HGF, bFGF, MCP-1 and IL-8, play important roles in cell survival and cell differentiation functions. Other trophic factors, such as BDNF and IL-6, have important roles in neural regeneration.

**EXAMPLE 8**

**Inhibition of IFN-gamma-Induced Expression of HLA-DR, DP, DQ on Expanded Human Umbilical Cord Tissue-Derived Cells by HMG-CoA Reductase Inhibitors**

Culture-expanded human umbilical cord tissue-derived cells (022803 P4) were seeded into 6-well tissue culture plates and cultured in Dulbecco's Modified Eagles Media (DMEM)-low glucose, 15% fetal bovine serum (FBS), penicillin/streptomycin (P/S), Betamercaptoethanol (BME) to approximately 70% confluence. The cells were then treated with media containing $10\mu M$ of respective HMG-CoA reductase inhibitor (Simvastatic acid (Alexis Biochemicals, Lausen, Switzerland) formulated as 10mM stock reagents in DMSO) or DMSO vehicle - 0.1% (Sigma, St. Louis, MO) and incubated overnight. The media was removed by aspiration and replaced with media containing 500U/ml rhIFN-gamma (BD Pharmingen, Franklin Lakes, NJ) and $10\mu M$ of respective HMG-CoA reductase inhibitor and incubated for 3 days. On day three, cells were harvested with trypsin.

Harvested cells were washed once with PBS and re-suspended in $100\mu l$ of 3% FBS in PBS with $20\mu l$ FITC-labeled HLA-DR,DP,DQ (BD Biosciences, Franklin Lakes, NJ) or FITC-labeled IgG antibody (BD Biosciences, Franklin Lakes, NJ) and incubated for one hour. Cells were washed once in PBS and resuspended in $500\mu l$ PBS and analyzed on a FACSCalibur flow cytometer (BS Biosciences, Franklin Lakes, NJ).

**Table 16. HLA-DR,DP,DQ expression of hUTC as measured by FITC fluorescence intensity values of pre-treated with HMG-CoA reductase inhibitor and further treated with inflammatory cytokine IFN-gamma.**

| HMG-CoA Reductase Inhibitor Treatment | IgG control | | IFN-gamma-treated | | No cytokine treatment | |
|---|---|---|---|---|---|---|
| | mean | Std dev | mean | Std dev | Mean | Std dev |
| Untreated | 4.88 | 5.12 | 274.23 | 219.04 | 5.56 | 8.97 |
| 0.1% DMSO vehicle control | 4.09 | 5.67 | 294.08 | 257.08 | 5.54 | 5.46 |
| Simvastatin | 4.4 | 2.38 | 5.57 | 3.98 | 5.66 | 3.25 |

As shown in Table 16, untreated and 0.1% DMSO vehicle control human umbilical cord tissue-derived cells incubated with the inflammatory cytokine IFN-gamma showed an increase in HLA-DR, DP, DQ expression as seen by increased fluorescence detected by flow cytometry. Human umbilical cord tissue-derived cells pre-treated with a HMG-CoA reductase inhibitor and subsequently incubated with IFN-gamma showed HLA-DR, DP, DQ expression similar to untreated and vehicle controls.

This data indicates that HMG-CoA reductase inhibits inflammatory cytokine-mediated expression of HLA-DR, DP, DQ in human umbilical cord tissue-derived cells.

**EXAMPLE 9**

**Efficacy of Human Umbilical Cord Tissue-Derived Cells (hUTC) in a Rabbit Model of Intervertebral Disc Degeneration**

A study was conducted to determine if human umbilical tissue-derived cells (hUTC) are effective in a rabbit model of Intervertebral Disc (IVD) degeneration. Cells were injected at the site of injury and disc dimensions evaluated by X-ray imaging. Analysis of the tissues was performed at necropsy.

To evaluate the effects of human umbilical cord tissue-derived cells (hUTC) on Intervertebral disc (IVD) degeneration, hUTC were injected into a punctured IVD. Bi-weekly X-rays were obtained and analyzed for change of disc height as compared to injured vehicle treated control. Treatment with hUTC resulted in increased disc-height as well as increased rate of recovery as compared to vehicle controls.

Creation of Disc Degeneration Model: Female NZW Rabbits at 6 months of age were selected without any systemic bias. Animals were tagged and weighed prior to enrollment and immediately prior to necropsy. Rabbits received glycopyrolate (0.01 to 0.02 mg/kg SQ) prior to sedation to reduce orotracheal secretions and lessen bradychardia associated with anesthesia. Buprenorphine (buprenorphine HCl 0.03 mg/kg) was given preoperatively as a preemptive analgesic. Rabbits were anesthetized by the administration of ketamine hydrochloride (25 mg/kg) and acepromazine maleate (1 mg/kg, 10 mg/ml) to facilitate endotracheal intubation. A pre-operative X-ray was taken as a baseline control. A dose of xylazine at 5 mg/kg was given subcutaneously or intramuscularly after the pre-op radiographs were completed. Animals were maintained by isoflurane inhalation (induced at 2-3%, and maintained at 0.5-2%).

The rabbits (weighing 3.5kg) were placed in a lateral prone position. Following prepping and draping, the lumbar IVDs were exposed through a posterolateral retroperitoneal approach by blunt dissection of the psoas muscle. The anterior surfaces of three consecutive lumbar IVDs (L2/3, L3/4 and L4/5) were exposed. Using an 18G needle with a stopper device that allows the needle to go to a depth of 5 mm, the annulus fibrosus was punctured in the ventral aspect into the nucleus pulposus at the L2/3 and L4/5 levels. A vascular staple and a suture were placed on the psoas muscle at the L3/4 level as markers. The surgical wound created was repaired in layers. The skin was closed using staples.

After the operation, a post-operative X-ray was taken to confirm the level of puncture. 1.5 mg of meloxicam was given orally (one day before surgery and 2-3 days after the operation). An analgesic (buprenorphine HCl 0.01 - 0.03 mg/kg) was given up to twice daily for 2-3 days, as needed, in consultation with the veterinary staff. After recovery from anesthesia, the rabbits were returned to their cages and mobilized ad lib. Rabbit jackets were used on rabbits to prevent them from reaching and disturbing/dehiscing the surgical incision and removing staples.

Evaluation of Treatments: Four weeks after the initial surgery (annular puncture), a similar surgical procedure was made from the opposite side to avoid bleeding from the scar formed from the first operation. Once the surgically degenerating discs were confirmed by X-ray and visual inspection, PBS, 1,000,000 or 100,000 cells were intradiscally injected into the nucleus pulposus area with a microsyringe using a 28G needle at both the L2/3 and L4/5 levels for each rabbit. L3/4 was left as unpunctured, untreated control. After repair of the surgical wound, the rabbits were returned to their cages and closely monitored. As previously described, an antibiotic and analgesic was administered for three days. Each rabbit received 1.5 mg of meloxicam orally (one day before surgery and 2-3 days after the operation). The behavior, appetite and change in body weight were closely monitored and the veterinary staff and the investigators monitored post-surgical stress.

All injection materials were prepared under sterile conditions. Research grade hUTC (Lot Q030306), evaluated for sterility, mycoplasma, karyotype, and pathogens were used for this study. Cryogenically stored cells were rapidly thawed and diluted in PBS. Cells were centrifuged and supernatant removed. Cells were resuspended in PBS and counted to achieve a final cell concentration of either 100,000 or 10,000 cells per micro liter. Trypan blue was used to assess viability. Ten microliters of cells were loaded into pre-sterilized micro-syringes and injected into the IVD as described above.

At 2-, 4-, 6-, 8-, 10-, 12-, 14- and 16- weeks post-puncture and sacrifice at 16-weeks post-puncture, X-rays to measure IVD height were taken after administration of ketamine hydrochloride (25 mg/kg) and acepromazine maleate (1 mg/kg).

At 16 weeks after the initial annular puncture (corresponding to weeks 12, after the injection (cells)), eight rabbits in each group were anesthetized with ketamine hydrochloride (25 mg/kg) and acepromazine maleate (1 mg/kg) and euthanized with an excess dose of pentobarbital (90 mg/kg, Euthanasia B solution: Henry Schein Inc., Melville, NY).

X-ray films obtained before the puncture, at each time point after the puncture, and at euthanasia were digitized and the vertebral body height and disc height were measured. The IVD height was expressed as the DHI according to published methods (Chujo et al., Spine, 2006; 31:2909-17; Masuda et al., Spine, 2006; 31:742-54). An orthopedic researcher, blinded to the treatment group, independently interpreted all X-ray images. Digitized X-rays, measurements, including the vertebral body height and IVD height, were analyzed using the custom program for MATLAB software (Natick, MA). Data were transported to Excel software and the IVD height was expressed as the disc height index (DHI = IVD height / adjacent IVD body height) based on the method of Lu et al., Spine, 22:1828-34 (1997), with a slight modification. The average IVD height (DHI) was calculated by averaging the measurements obtained from the anterior, middle and posterior portions of the IVD and dividing that by the average of adjacent vertebral body heights. %DHI was expressed as (postoperative DHI/preoperative DHI) x 100. Furthermore, %DHI was normalized using the L3/4 level as the control level. The normalized %DHI = (experimental level %DHI / L3/4 %DHI) x 100. The recovery rate was also calculated as follows: (%DHI (time point) - %DHI (4W [puncture])) / (100 - %DHI (4W [puncture])).

The significance of differences among means of data on X-ray measurements was analyzed by two-way repeated measures ANOVA, or one-way ANOVA and Fisher's PLSD as a post hoc test. All data are expressed as the mean $\pm$ standard error. Statistical analysis was performed using the Statview (Version 5.0, SPSS, Chicago, IL) program package with a significance level of $p < 0.05$.

Disc height index was assessed biweekly as described above. Discs with less than a 10% deficit were excluded from the study. Data (shown in Table 17) indicate the disc height was increased with an hUTC dose of 100,000 cells and decreased with a dose of 1,000,000 cells as compared to control between 2-12 weeks post-transplant (6-16 weeks post-puncture) ($p < 0.05$ hUTC (1,000,000) vs. hUTC (100,000) 2, 4, 6, and 14 weeks post-transplant; $p < 0.01$ 8 weeks post-transplant).

| Table 17. Effect of hUTC on Disc Height | | | | | | | | | |
|---|---|---|---|---|---|---|---|---|---|
| Disc Height (%) | 0 Pre-OP | 2 2W-P | 4 4W-P | 6 2W | 8 4W | 10 6W | 12 8W | 14 10W | 16 12W |
| Control | 100.0 | 79.4 | 79.3 | 78.9 | 79.5 | 78.5 | 77.1 | 74.8 | 78.5 |
| Control- SE | 0.0 | 3.2 | 2.8 | 2.1 | 2.6 | 3.4 | 4.6 | 3.6 | 2.0 |
| 1.00E+06 | 100.0 | 76.6 | 76.6 | 74.8 | 74.6 | 72.7 | 70.8 | 74.4 | 72.9 |
| SE | 0.0 | 2.2 | 1.5 | 2.1 | 1.8 | 2.1 | 1.4 | 1.8 | 2.8 |
| 1.00E+05 | 100.0 | 84.8 | 76.6 | 83.7 | 83.8 | 81.3 | 86.0 | 82.4 | 82.2 |
| SE | 0.0 | 1.7 | 2.6 | 2.7 | 3.3 | 1.9 | 3.1 | 4.2 | 3.8 |

Recovery rate was measured as described above. Discs with less than a 10% deficit were excluded from the study. Data (shown in Table 18) indicate the recovery rate was increased with an hUTC dose of 100,000 and decreased with a dose of 1,000,000 as compared to control between 2-12 weeks post-transplant (6-16 weeks post-puncture). ($p < 0.05$ Control vs. hUTC (100,000 cells) 2 and 14 weeks post-transplant; $p < 0.01$ 12 weeks post-transplant).

| Table 18. Effect of hUTC on Recovery Rate (%) | | | | | | | | | |
|---|---|---|---|---|---|---|---|---|---|
| Recovery Rate | 0 Pre-OP | 2 2W-P | 4 4W-P | 6 2W | 8 4W | 10 6W | 12 8W | 14 10W | 16 12W |
| Control- | 0.0 | 0.0 | 0.0 | -0.1 | -0.1 | -0.1 | -0.1 | -0.3 | -0.2 |
| Control- SE | 0.0 | 0.0 | 0.0 | 0.1 | 0.2 | 0.2 | 0.3 | 0.2 | 0.2 |
| 1.00E+06% | 0.0 | 0.0 | 0.0 | -0.1 | -0.1 | -0.2 | -0.3 | -0.1 | -0.2 |
| SE | 0.0 | 0.0 | 0.0 | 0.1 | 0.1 | 0.1 | 0.1 | 0.1 | 0.1 |
| 1.00E+05% | 0.0 | 0.0 | 0.0 | 0.3 | 0.2 | 0.1 | 0.4 | 0.3 | 0.3 |
| SE | 0.0 | 0.0 | 0.0 | 0.1 | 0.2 | 0.1 | 0.2 | 0.1 | 0.1 |

Thus, the effects of hUTC on IVD degeneration were evaluated in a rabbit model of IVD degeneration by injecting hUTC into a punctured IVD at dose of 1,000,000 and 100,000 cells/injection. Bi-weekly X-rays were obtained and analyzed for change of disc height as compared to untreated punctured control. Data indicated that treatment with hUTC at a concentration of 100,000 resulted in increased disc-height, where treatment with a concentration of 1,000,000 decreased disc height as compared to PBS-treated control (Table 17). Further analysis of the data revealed that the rate of recovery (recovery amount/total decrease of normalized %DHI) of discs treated with hUTC (100,000) was higher than that of control (Table 18).

## EXAMPLE 10

### Expression of Extracellular Matrix Proteins by Human Umbilical Cord Tissue-Derived -Cells *in vitro*

A study was conducted to determine the degree of expression of the three extracellular matrix proteins aggrecan, collagen I and collagen II by hUTC *in vitro.* Cells were tested alone and after stimulation with the trophic factors, TGF-beta, GDF-5 and PDGF-BB.

Cultures of human umbilical tissue-derived cells (hUTC; lot 120304) were maintained in culture under standard conditions. In brief, cells were seeded at 5000 cells per square cm in T-flasks with passage and reseeding every 3-4 days. Cells for the trophic factor experiment were encapsulated in alginate beads and treated with factors to the standard growth media. Cultures were supplemented with ascorbic acid at 100 μg/ml. Factors tested were PDGF-BB at 10 ng/ml, TGFbeta-1 at 5 ng/ml and GDF-5 at 200 ng/ml. Five treatment groups were created, hUTC alone, hUTC with GDF-5, hUTC with TGF-beta1, hUTC with PDGF-BB and hUTC with TGF-beta1 and GDF-5.

After 2 weeks in culture, cells were released from alginate, washed, pelleted and frozen. RNA was isolated and reverse transcription performed to generate cDNA. Samples were analyzed by real-time PCR for the expression of aggrecan, collagen type I and type II.

The results from the real-time PCR analysis show the expression under five different culture conditions. Results are shown in Table 19 and expressed as relative expression to hUTC without growth factor treatment. The cultures treated with PDGF-BB and GDF-5 with TGF-beta1 showed comparable expression of aggrecan, collagen I and collagen II. The cells treated with TGF-beta1 showed some induction of collagen I and collagen II and aggrecan of approximately 10-20 fold. The highest induction was observed with GDF-5 treatment. The cells showed approximately a 50-fold increase in aggrecan and collagen type I and a more than 300 fold increase in collagen type II expression.

| Table 19. Expression of Extracellular Matrix Proteins by hUTC *in vitro.* | | | |
|---|---|---|---|
| | Relative mRNA | | |
| | Aggrecan | Collagen I | Collagen II |
| hUTC | 1 | 1 | 1 |
| hUTC + GDF-5 | 56 | 45 | 387 |
| hUTC + GDF-5/TGF beta | 1 | 1 | 113 |
| hUTC + PDGF | 1 | <1 | <1 |
| hUTC +TGF beta | 23 | 7 | 13 |

**EXAMPLE 11**

**Telomerase Expression in umbilical-derived cells**

Telomerase functions to synthesize telomere repeats that serve to protect the integrity of chromosomes and to prolong the replicative life span of cells (Liu, K, et al., PNAS, 1999; 96:5147-5152). Telomerase consists of two components, telomerase RNA template (hTER) and telomerase reverse transcriptase (hTER). Regulation of telomerase is determined by transcription of hTERT but not hTERT. Real-time polymerase chain reaction (PCR) for hTERT mRNA thus is an accepted method for determining telomerase activity of cells.

**Cell Isolation.** Real-time PCR experiments were performed to determine telomerase production of human umbilical cord tissue-derived cells. Human umbilical cord tissue-derived cells were prepared in accordance the examples set forth above. Generally, umbilical cords obtained from National Disease Research Interchange (Philadelphia, Pa.) following a normal delivery were washed to remove blood and debris and mechanically dissociated. The tissue was then incubated with digestion enzymes including collagenase, dispase and hyaluronidase in culture medium at 37°C. Human umbilical cord tissue-derived cells were cultured according to the methods set forth in the examples above. Mesenchymal stem cells and normal dermal skin fibroblasts (cc-2509 lot # 9F0844) were obtained from Cambrex, Walkersville, Md. A pluripotent human testicular embryonal carcinoma (teratoma) cell line nTera-2 cells (NTERA-2 cl.DI), (*see*, Plaia et al., Stem Cells, 2006; 24(3):531-546) was purchased from ATCC (Manassas, Va.) and was cultured according to the methods set forth above.

**Total RNA Isolation.** RNA was extracted from the cells using RNeasy® kit (Qiagen, Valencia, Ca.). RNA was eluted with 50 microliters DEPC-treated water and stored at -80°C. RNA was reverse transcribed using random hexamers with the TaqMan® reverse transcription reagents (Applied Biosystems, Foster City, Ca.) at 25°C for 10 minutes, 37°C for 60 minutes and 95°C for 10 minutes. Samples were stored at -20°C.

**Real-time PCR.** PCR was performed on cDNA samples using the Applied Biosystems Assays-On-Demand™ (also known as TaqMan® Gene Expression Assays) according to the manufacturer's specifications (Applied Biosystems). This commercial kit is widely used to assay for telomerase in human cells. Briefly, hTERT (human telomerase gene) (HS00162669) and human GAPDH (an internal control) were mixed with cDNA and TaqMan® Universal PCR master mix using a 7000 sequence detection system with ABI prism 7000 SDS software (Applied Biosystems). Thermal cycle conditions were initially 50°C for 2 minutes and 95°C for 10 minutes followed by 40 cycles of 95°C for 15 seconds and 60°C for 1 minute. PCR data was analyzed according to the manufacturer's specifications.

Human umbilical cord tissue-derived cells (ATCC Accession No. PTA-6067), fibroblasts, and mesenchymal stem cells were assayed for hTERT and 18S RNA. As shown in Table 20, hTERT, and hence telomerase, was not detected in human umbilical cord tissue-derived cells.

| Table 20 | | |
|---|---|---|
| | hTERT | 18S RNA |
| Umbilical cells (022803) | ND | + |

(continued)

| Table 20 | | |
|---|---|---|
| | hTERT | 18S RNA |
| Fibroblasts | ND | + |
| ND- not detected; + signal detected | | |

Human umbilical cord tissue-derived cells (isolate 022803, ATCC Accession No. PTA-6067) and nTera-2 cells were assayed and the results showed no expression of the telomerase in two lots of human umbilical cord tissue-derived cells while the teratoma cell line revealed high level of expression (Table 21).

| Table 21 | | | | | |
|---|---|---|---|---|---|
| Cell type | hTERT | | GAPDH | | hTERT norm |
| | Exp. 1 | Exp. 2 | Exp. 1 | Exp. 2 | |
| nTera2 | 22.85 | 27.31 | 16.41 | 16.31 | 0.61 |
| 022803 | - | - | 22.97 | 22.79 | - |

Therefore, it can be concluded that the human umbilical tissue-derived cells of the present invention do not express telomerase.

## EXAMPLE 12

### Injection of Human Umbilical-Tissue Derived Cells into the Nucleus Pulposus Alters the Course of Intervertebral Disc Degeneration *in vivo*

This Example investigates the utility of injecting human umbilical-tissue derived cells (hUTC) directly into the Nucleus Pulpous (NP) in a surgical *in vivo* model of IVD degeneration. The hUTC were injected with and without a hydrogel carrier. This study is among the first to investigate MRI and biomechanical responses to treatment in this degeneration model.

### Methods

As described more in detail below, thirty skeletally mature New Zealand White rabbits were used (control n=6, puncture n=6, hydrogel carrier n=6, cells + PBS buffer n=6, cells + hydrogel carrier n=6) in a previously validated rabbit annulotomy model for intervertebral disc degeneration *(see* Sobajima et al. Spine. 2005; 30(1): 15-24). Discs L2-3, L3-4, and L4-5 were punctured were punctured with a 16G needle to induce degeneration and then subsequently treated with human umbilical-tissue derived cells with or without a hydrogel carrier. Serial spine MRIs obtained at 0, 3, 6, and 12 weeks using a 3T knee coil were analyzed for evidence of degeneration according to previously validated methodology. The rabbits were sacrificed at 12 weeks and discs L4-5 were analyzed histologically. Viscoelastic properties of the L3-4 discs were analyzed using uniaxial load-normalized displacement testing. Creep curves were mathematically modeled according to a previously validated two-phase exponential model.

### Rabbits

Thirty healthy skeletally mature New Zealand White rabbits were used in this study (female, age 1 year, weight 5kg). They were split into non-punctured control (n=6), puncture (n=6), puncture followed by injection of carrier alone (n=6), puncture followed by injection of cells in PBS buffer (n=6), and puncture followed by injection of cells in carrier (n=6).

### Sample Preparation

Human umbilical cord tissue was obtained from a consented donor undergoing either vaginal or cesarean delivery. Human umbilical tissue-derived cells (hUTC) were isolated and expanded from the umbilical cord of a single donor. Briefly, the umbilical cord was manually minced and digested with a mixture of 0.5 Units/ml collagenase (Nordmark), 5.0 Units/ml, dispase (Roche Diagnostics), and 2 Units/ml hyaluronidase (ISTA Pharmaceuticals) until almost completely digested. The cell suspension was passed through a sieve to remove undigested tissue, and the cells were centrifuged and seeded at 5,000 cells per $cm^2$ on porcine gelatin-coated flasks in Growth Media 1 (DMEM-low glucose (Cambrex/SAFC Biosciences), 15% (vol/vol) defined fetal bovine serum (FBS; HyClone, Logan, UT), 0.001% betamercaptoethanol (Sigma), 50 U/ml penicillin and 50 $\mu$g/ml streptomycin (Lonza)). Cells were expanded in static T-flasks, under standard tissue culture conditions in atmospheric oxygen with 5% carbon dioxide at 37° until reaching five population doublings (PD) and were banked. The cells were than expanded in static T-flasks to approximately 12 cumulative PD in Growth Media 2 (DMEM-low glucose, 15% (vol/vol) defined fetal bovine serum (FBS; HyClone,), 100 U/ml penicillin, and 100 $\mu$g/ml streptomycin (Invitrogen)) and were banked. Cells were then further expanded on HILLEX® II microcarriers (SoloHill Engineering) in spinner flasks (Corning, NY) in Growth Media 2. The spinner flasks containing cells were placed on spinner plates set to 60 rpm and the cells were cultured under standard conditions in atmospheric oxygen with 5% carbon dioxide at 37°C until they reached approximately 25-30 total cumulative population doublings. Cells were harvested from microcarriers using TrypLE™ (Invitrogen), cryopreserved and stored at cryogenic temperatures. Aliquots of the cell bank were thawed for characterization testing that included viability, recovery, sterility, endotoxin, mycoplasma, karyology and cell surface marker immunophenotyping to ensure safety and identity. Cells were tested at an early passage for viral pathogens by a PCR-based method to detect nucleic acid sequences specific to HIV-1, HIV-2, CMV, HBV, HCV, HTLV and EBV. Cells were stored at cryogenic temperatures until use and prepared for injection immediately prior to delivery. Immediately prior to injections to the animals, cell vials were thawed in a 37°C water bath, and then washed with PBS. Aliquots of the cell suspension were removed and mixed with Trypan blue and counted with a hemocytometer. The cell concentration was adjusted to the appropriate density for delivery. The carrier solution was either PBS or an *in situ* forming hydrogel carrier derived from the components of EVICEL® fibrin glue (EVICEL® Fibrin sealant (Human), Omrix Pharmaceuticals, Ltd.). To load cells into the hydrogel, the cells were mixed with human fibrinogen (6.8-10.6 mg/ml) in PBS. This reagent was mixed with human thrombin (0.4-0.6 U/ml) in PBS. Gelation occurred shortly after mixing.

## Puncture Surgery

In a veterinary operating room, under sterile surgical conditions and general anesthesia, the rabbits' lumbar spines were exposed from a left antero-lateral approach. The L4-5 intervertebral disc was identified based on its position relative to the iliac crest, and was punctured with a 16 gauge needle to a depth of 5 mm such that the tip of the needle was in the center of the disc. The needle entered the disc parallel to the endplates with the bevel pointed cephalad. Discs L3-4 and L2-3 were subsequently identified by direct visualization and palpation, and punctured as above. After surgery, the rabbits were housed in large private cages that allowed unrestricted movement. Annulotomy of a rabbit's IVD by this method induces a slow, reliable, reproducible cascade of degeneration, demonstrable by serial T2 weighted MRIs (*see* Masuda et al. Spine. 2005; 30(1):5-14).

## Injection Surgery

The rabbits in the treatment groups returned to surgery 3 weeks after initial puncture surgery. The spine was exposed from a right anterolateral approach (contralateral to the prior puncture surgery). A Hamilton 100 microliter syringe (1710TPLT; product #81041) and a Hamilton 30 gauge sharp tipped needle with a plastic hub (KF 730 NDL 6/pack, 30G/2"; product # 90130) were used for therapeutic injections into the center of the NP. For the carrier group, 15 microliters of hydrogel was drawn up into the syringe, and then slowly injected into each disc (in less than 4.5 minutes to facilitate liquid injection prior to gelling). For the cells + buffer (Phosphate Buffered Saline) group, $10^5$ cells in 15 microliters of sterile PBS were injected into each disc. For the cells in carrier group, $10^5$ cells in 15 microliters of hydrogel carrier were injected. Injections were performed under c-arm guidance to confirm that the needle was in the center of the disc. All injections were performed at a deliberately slow and constant rate over the course of one minute to avoid rapid increases in disc pressure and subsequent extrusion of materials. The rabbits were closed, revived, and cared for as described for the puncture surgery.

## Magnetic Resonance Imaging

Sagittal MRIs were obtained at time 0 (prior to annular puncture), 3 weeks (prior to injection surgery), 6 weeks, and 12 weeks (prior to sacrifice). A 3 Tesla Siemens magnet and standard human knee coil were used to obtain T1 weighted images (TR = 650 ms, TE = 14 ms, slice thickness = 0.6 mm) and T2 weighted images (TR = 3800 ms, TE = 114 ms, slice thickness = 0.6 mm). The rabbits were sedated and placed in the knee coil in the supine position. The T1 weighted images were used to qualitatively check for any bone abnormalities in the spine. The T2 weighted images were used to quantify the amount of degeneration in the discs. The mid-sagittal slice of the T2 weighted sequences was identified by a trained physician based on the width of the vertebral body, spinal cord, and spinous processes. A previously validated automated segmentation method was employed to identify the NP as the region of interest *(see* Bechara et al. Am J Neuroradiology. 2010; 31(9):1640-1644). The MRI index was calculated as the sum of pixel area multiplied by pixel intensity for each pixel within the region of interest. The percentage NP area and MRI index relative to control values at week 0 were averaged across the three discs of interest and plotted against time points. Statistical significance was calculated with a student's T-test ($p < 0.05$).

## Sacrifice and Sample Processing

All rabbits were sacrificed 12 weeks after the initial puncture surgery (after the final MRI was obtained). Immediately after death, the spines were dissected out *en block.* Disc L4-5 was prepared for histological analysis. The discs were fixed and decalcified with DECALCIFIER I® (Surgipath Medical Indus., Inc.) for two weeks, then dehydrated in histology tissue processor and embedded in paraffin. Next, the discs were sectioned at a thickness of 5 $\mu$m in the sagittal plane. The sections were stained with Hematoxylin and Eosin (H&E) (Sigma) using standard histology protocol, and photographed using a Nikon E800 microscope.

## Biomechanics

After sacrifice, disc L3-4 was dissected out as a functional spinal unit (FSU, bone-disc-bone) for biomechanical analysis. Samples were cleaned of posterior elements, potted in epoxy resin, and mounted with custom fixtures in an axial testing machine controlled with a Matlab (Matlab R2008a, The Mathworks, Inc., Natick, MA) fuzzy logic program. All discs were wrapped in saline-soaked gauze to minimize dehydration, and testing was performed on the same day as sacrifice. Specimens were preconditioned with 20 cycles of compressive loading (0 to 1.0 MPa, 0.1 mm/s) and then subjected to constant compression (1.0 MPa) for 1100 seconds. This load target was selected because it is comparable to pressures experienced by the human IVD in activities of daily living (adjusted for the smaller size of the rabbit discs) (Wilke et al. Spine. 1999; 24(8):755-762). The initial ramp phase was defined as the first 200 microseconds of testing, while the creep phase was defined as the remainder of testing. Following testing, creep curves were fitted with a two-phase exponential model,

$$\frac{d(t)}{L_0} = \frac{1}{S_1}\left(1 - e^{-S_1 t/\eta_1}\right) + \frac{1}{S_2}\left(1 - e^{-S_2 t/\eta_2}\right)$$

where *d(t)* is the axial displacement over time, $L_o$ is the applied axial load, $S_1$ and $S_2$ are elastic damping coefficients (N/mm), and $\eta_1$ and $\eta_2$ are viscous damping coefficients (Ns/mm). Average curves were generated and fitted with the exponential model for each condition.

## Results

## Rabbits

No adverse effects were observed in any groups as a result of treatment.

## MRIs

The T1 weighted midsagittal MRIs of the L2-3, L3-4, and L4-5 discs demonstrated no significant changes or osteophyte formation. The T2 weighted midsagittal MRIs of the discs in the punctured group degenerated (the ROI darkened and collapsed from 0 to 12 weeks), compared to non-punctured controls. All three treatment groups demonstrated less degeneration, qualitatively, than the punctured group as shown in Figures 1 and 2. The punctured (and not treated) discs showed the most degeneration with a decrease of 59% in NP area and a decrease of 64% in MRI Index across time-points. All three treatment groups demonstrated less degeneration based on both total NP area and MRI Index than the punctured group. The carrier + cells group had the least decrease in area and MRI Index among the treated groups and most closely resembled the control group (Figure 3). At 0 and 3 weeks there were no statistically significant differences in MRI Index or area between control vs. any of the three treatment groups and puncture, or between puncture vs. treatment groups. At 6 and 12 weeks the differences in both MRI Index and area between control and puncture was statistically significant, as would be expected of a valid degeneration model. The control and carrier groups showed statistically significant differences in MRI Index at 6 and 12 weeks, and in area at 12 weeks. The control and buffer + cells groups were statistically significantly different in MRI index at 12 weeks. The control and carrier + cells groups were statistically significantly different in both MRI Index and area at both 6 and 12 weeks. The puncture and carrier groups showed no statistically significant differences in MRI quantification across time-points. The puncture and buffer + cells groups were statistically significantly different in both MRI Index and area at both 6 and 12 weeks. The puncture and carrier + cells groups were significantly different in MRI Index and area at 12 weeks.

**Biomechanics**

Curves representing total load-normalized displacements (initial ramp phase and subsequent creep phase) are given in Figure 4. There is a trend toward differences among groups. Specifically, the curve generated by the control groups is similar to the carrier + cells group, the punctured group is similar to the buffer + cells group, and the carrier group lies between these groups. Most of the variability between groups was from the initial ramp phase. Despite these trends, when the creep phase of the curve was fit with a two-phase exponential model, the early and late viscous and elastic damping coefficients did not yield any statistically significant differences (data not shown).

**Histology**

Representative sagittal cuts of disc L4-5 were stained with Hematoxylin and Eosin (H&E) and inspected at 20x and 100x magnification (Figures 5, 6 and 7). Control discs maintained their architecture. As expected, punctured discs demonstrated loss of cellularity and fibrosis in the NP, suggestive of degeneration. Punctured discs treated with the hydrogel carrier resulted in some loss of cellularity but maintained a robust extracellular matrix. Treatment with cells + buffer demonstrated relative preservation of NP area, although significant fibrosis was observed. Treatment with cells + carrier resulted in improved cellularity and architecture compared to punctured discs.

**Discussion**

This example contains a broad and diverse range of outcome measures, including novel MRI technology and biomechanics, to not only quantify invertertebral disc degeneration (IDD), but also to show a response to a targeted treatment for disc degeneration. As discussed in detail in this example, the treatment groups demonstrated viscoelastic properties that were distinct from control and punctured values.

Based on MRI, histological, and biomechanical criteria, injection of rabbit lumbar intertebral discs (IVDs) undergoing IDD with hUTC in a carrier has a beneficial effect compared to either carrier alone or cells + buffer alone. All three treatments appeared beneficial compared to untreated punctured discs. On MRI, the nucleus pulposus (NP) area and index for all three treatment groups was in between the control (non-degenerated), and punctured (most degenerated) conditions, indicating that treatment helped partially delay disc height and signal intensity changes. Axial loading generated displacement curves that were distinct appearing for each condition. Histology showed variable restoration of disc architecture and cellularity with treatment.

The cells + PBS group generated MRI data that fell between control and punctured values, and was very similar appearing to the carrier group's MRI data. However, this group's displacement curve was most similar to the punctured state, suggesting that although this treatment helped partially restore NP area and signal intensity on MRI, it was not very effective at restoring the disc's native mechanical properties.

The carrier alone group generated MRI data that fell between punctured and non-punctured values. In the instant example, the displacement curve for the hydrogel group also fell between the punctured and non-punctured state, indicating that the hydrogel carrier was capable of restoring some mechanical properties to the disc independent of any extracellular matrix synthesis by native or transplanted cells. Histology from the carrier group does have more cellularity than might be expected from a non-cellular treatment, possibly indicating that the hydrogel provided a safe haven in which native cells could thrive.

The cells + carrier group incorporated both the cell transplant and the hydrogel injection of the other two groups. This group had a consistent but non-significant trend toward MRI degenerative measures that were better than the other two treatment groups (most similar to the control state, although still significantly distinct from control values). This group also had a displacement curve that was most similar to the control curve, suggesting the best mechanical response. The histology from this group had a notable increase in cellularity compared to the punctured group and a relative preservation in architecture, but there is evidence of fibrosis.

There was no histological evidence of an immune response generated from transplanting human cells into a rabbit disc. None of the treated rabbits exhibited signs of illness, and there was no histological evidence of an inflammatory response.

Human postpartum umbilical tissue is an appealing source for therapeutic cells as donor tissue is readily available and cells are easily harvested without the ethical conflicts that are associated with embryonic stem or fetal cells.

In summary, the punctured group underwent MRI and histological evidence of degeneration compared to the non-punctured controls, as expected. The treatment groups underwent less MRI and histological evidence of degeneration than the punctured group. The treatment groups demonstrated viscoelastic properties that were distinct from control and punctured values. This study shows that injection of punctured rabbit lumbar IVDs undergoing degeneration with (a) hUTC and (b) hydrogel slows the course of IVD degeneration. Injection of the cells in hydrogel slows the progression of degeneration more than either (a) hUTC alone or (b) hydrogel alone. Thus, this study demonstrates that the treatment of punctured rabbit intervertebral discs with human umbilical-tissue derived cells (hUTC), with and without a hydrogel carrier solution, helps restore MRI, histological, and biomechanical properties to values near those of non-punctured controls.

**Claims**

1. A hydrogel and an isolated homogenous population of cells obtained from human umbilical cord tissue, or a pharmaceutical composition comprising said hydrogel and isolated homogenous population of cells, for use in treating a disease or condition related to intervertebral disc degeneration, wherein the treating comprises improving cellularity and architecture of a degenerated disc,
wherein the umbilical cord tissue is substantially free of blood and the isolated homogenous population of cells is capable of self-renewal and expansion in culture, has the potential to differentiate and does not express CD117 or telomerase.

2. The hydrogel and population of cells or pharmaceutical composition for the use of claim 1, wherein the isolated population of cells has one or more of the following characteristics:

   (a) expresses reticulon, chemokine receptor ligand 3, and/or granulocyte chemotactic protein;
   (b) does not produce CD31, CD34 and HLA-DR;
   (c) expresses, relative to a human fibroblast, mesenchymal stem cell, or iliac crest bone marrow cell, increased levels of interleukin 8 and reticulon 1; and
   (d) expresses CD10, CD13, CD44, CD73, and CD90.

3. The hydrogel and population of cells or pharmaceutical composition for the use of claim 1 or claim 2, wherein the treating comprises administering the pharmaceutical composition by injection.

4. The pharmaceutical composition for the use of claim 1, wherein the pharmaceutical composition further comprises at least one other cell type and/or at least one agent.

**5.** The hydrogel and population of cells or pharmaceutical composition for the use of any one of the preceding claims, wherein the treating comprises administering the hydrogel and population of cells or pharmaceutical composition into a degenerated intervertebral disc, optionally into the nucleus pulposus or into the annulus fibrosus of the intervertebral disc.

**6.** The hydrogel and population of cells or pharmaceutical composition for the use of any one of the preceding claims, further comprising inducing the isolated homogenous population of cells obtained from human umbilical cord tissue to at least partially differentiate *in vitro,* wherein optionally the isolated homogenous population of cells is induced to differentiate into cells displaying an annulus fibrosus cell phenotype or a nucleus pulposus cell phenotype.

**7.** The hydrogel and population of cells for the use of any one of the preceding claims, wherein treating comprises administering the hydrogel simultaneously with, or before, or after, the isolated homogenous population of cells obtained from human umbilical cord tissue.

**8.** The hydrogel and population of cells for the use of any one of the preceding claims, wherein the treating comprises administering the isolated homogenous population of cells within an implantable device.

**9.** The hydrogel and population of cells for the use of any one of the preceding claims, wherein the treating further comprises administering at least one other cell type simultaneously with, or before, or after, the isolated homogenous population of cells obtained from human umbilical cord tissue.

**10.** The hydrogel and population of cells or pharmaceutical composition for the use of claim 4 or 9, wherein the at least one other cell type is engineered to express at least one exogenous gene product, wherein optionally the exogenous gene product is a trophic factor.

**11.** The hydrogel and population of cells for the use of claim 10, wherein the exogenous gene product modulates expression of one or more extracellular matrix proteins.

**12.** The hydrogel and population of cells for the use of any one of the preceding claims, wherein the treating further comprises administration of at least one agent.

**13.** The hydrogel and population of cells or pharmaceutical composition for the use of claim 4 or claim 12, wherein the at least one agent is a trophic factor, optionally selected from the group consisting of TGF-beta, GDF-5, PDGF-BB and TIMP1, wherein optionally the trophic factor exerts a trophic effect on the isolated homogenous cell population obtained from human umbilical cord tissue.

**14.** The hydrogel and population of cells or pharmaceutical composition for the use of claim 1, wherein the hydrogel comprises fibrinogen and thrombin and/or fibrin glue.

**Patentansprüche**

**1.** Hydrogel und aus menschlichem Nabelschnurgewebe erhaltene isolierte homogene Zellpopulation oder pharmazeutische Zusammensetzung, die das Hydrogel und die isolierte homogene Zellpopulation umfasst, zur Verwendung bei der Behandlung einer Krankheit oder eines Leidens, das mit Bandscheibendegeneration in Zusammenhang steht, wobei das Behandeln das Verbessern der Zellularität und der Architektur der degenerierten Bandscheibe umfasst,
wobei das Nabelschnurgewebe im Wesentlichen frei von Blut ist, und die isolierte homogene Zellpopulation zur Selbsterneuerung und Vermehrung in Kultur imstande ist, das Potential hat, sich zu differenzieren und keine CD117 oder Telomerase exprimiert.

**2.** Hydrogel und Zellpopulation oder pharmazeutische Zusammensetzung zur Verwendung nach Anspruch 1, wobei die isolierte Zellpopulation eine oder mehr der folgenden Eigenschaften aufweist:

(a) sie exprimiert Reticulon, Chemokinrezeptorligand 3, und/oder chemotaktisches Granulocytenprotein;
(b) sie produziert kein CD31, CD34 und HLA-DR;
(c) sie exprimiert im Vergleich zu einem menschlichen Fibroblasten, einer mesenchymalen Stammzelle oder einer Beckenkamm-Knochenmarkzelle erhöhte Mengen an Interleukin 8 und Reticulon 1; und

(d) sie exprimiert CD10, CD13, CD44, CD73 und CD90.

3. Hydrogel und Zellpopulation oder pharmazeutische Zusammensetzung zur Verwendung nach Anspruch 1 oder Anspruch 2, wobei das Behandeln das Verabreichen der pharmazeutischen Zusammensetzung durch Injektion umfasst.

4. Pharmazeutische Zusammensetzung zur Verwendung nach Anspruch 1, wobei die pharmazeutische Zusammensetzung zudem mindestens einen anderen Zelltyp und/oder mindestens ein Mittel umfasst.

5. Hydrogel und Zellpopulation oder pharmazeutische Zusammensetzung zur Verwendung nach einem der vorhergehenden Ansprüche, wobei das Behandeln das Verabreichen des Hydrogels und der Zellpopulation oder der pharmazeutischen Zusammensetzung in eine degenerierte Bandscheibe, gegebenenfalls in den Nucleus pulposus oder in den Annulus fibrosus der Bandscheibe, umfasst.

6. Hydrogel und Zellpopulation oder pharmazeutische Zusammensetzung zur Verwendung nach einem der vorhergehenden Ansprüche, zudem umfassend das Induzieren der aus dem menschlichen Nabelschnurgewebe erhaltenen isolierten homogenen Zellpopulation, damit diese sich zumindest partiell *in vitro* differenziert, wobei gegebenenfalls die isolierte homogene Zellpopulation induziert wird, damit sie sich in Zellen differenziert, die einen Annulus-fibrosus-Zellphänotyp oder einen Nucleus-pulposus-Zellphänotyp zeigen.

7. Hydrogel und Zellpopulation zur Verwendung nach einem der vorhergehenden Ansprüche, wobei das Behandeln das Verabreichen des Hydrogels zeitgleich mit oder vor oder nach der aus dem menschlichen Nabelschnurgewebe erhaltenen isolierten homogenen Zellpopulation umfasst.

8. Hydrogel und Zellpopulation zur Verwendung nach einem der vorhergehenden Ansprüche, wobei das Behandeln das Verabreichen der isolierten homogenen Zellpopulation in einer implantierbaren Vorrichtung umfasst.

9. Hydrogel und Zellpopulation zur Verwendung nach einem der vorhergehenden Ansprüche, wobei das Behandeln zudem das Verabreichen von mindestens einem anderen Zelltyp zeitgleich mit oder vor oder nach der aus menschlichem Nabelschnurgewebe erhaltenen isolierten homogenen Zellpopulation umfasst.

10. Hydrogel und Zellpopulation oder pharmazeutische Zusammensetzung zur Verwendung nach Anspruch 4 oder 9, wobei der mindestens eine andere Zelltyp derart gentechnisch verändert wurde, dass mindestens ein exogenes Genprodukt exprimiert wird, wobei gegebenenfalls das exogene Genprodukt ein trophischer Faktor ist.

11. Hydrogel und Zellpopulation zur Verwendung nach Anspruch 10, wobei das exogene Genprodukt die Expression von einem oder mehr extrazellulären Matrixproteinen moduliert.

12. Hydrogel und Zellpopulation zur Verwendung nach einem der vorhergehenden Ansprüche, wobei das Behandeln zudem die Verabreichung von mindestens einem Mittel umfasst.

13. Hydrogel und Zellpopulation oder pharmazeutische Zusammensetzung zur Verwendung nach Anspruch 4 oder Anspruch 12, wobei das mindestens eine Mittel ein trophischer Faktor ist, der gegebenenfalls ausgewählt ist aus der Gruppe, bestehend aus TGF-beta, GDF-5, PDGF-BB und TIMP1, wobei gegebenenfalls der trophische Faktor eine trophische Wirkung auf die aus menschlichem Nabelschnurgewebe erhaltene isolierte homogene Zellpopulation ausübt.

14. Hydrogel und Zellpopulation oder pharmazeutische Zusammensetzung zur Verwendung nach Anspruch 1, wobei das Hydrogel Fibrinogen und Thrombin und/oder Fibrinkleber umfasst.

**Revendications**

1. Hydrogel et population homogène isolée de cellules obtenues à partir de tissu de cordon ombilical humain, ou composition pharmaceutique comprenant lesdits hydrogel et population homogène isolée de cellules, pour utilisation dans le traitement d'une maladie ou d'une affection liée à une dégénérescence de disque intervertébral, le traitement comprenant l'amélioration de la cellularité et de l'architecture d'un disque dégénéré,
le tissu de cordon ombilical étant sensiblement exempt de sang et la population homogène isolée de cellules étant capable d'autorenouvellement et d'expansion en culture, et ayant le potentiel pour se différencier et n'exprimant pas CD117 ou la télomérase.

2. Hydrogel et population de cellules ou composition pharmaceutique pour l'utilisation de la revendication 1, où la population isolée de cellules possède une ou plusieurs des caractéristiques suivantes :

(a) exprime le réticulon, le ligand de récepteur de chimiokine 3, et/ou la protéine chimiotactique de granulocyte ;
(b) ne produit pas CD31, CD34 et HLA-DR ;
(c) exprime, par rapport à un fibroblaste, une cellule souche mésenchymateuse ou une cellule de moelle osseuse de crête iliaque humaine, des taux augmentés d'interleukine 8 et de réticulon 1 ; et
(d) exprime CD10, CD13, CD44, CD73, et CD90.

3. Hydrogel et population de cellules ou composition pharmaceutique pour l'utilisation de la revendication 1 ou la revendication 2, le traitement comprenant l'administration de la composition pharmaceutique par injection.

4. Composition pharmaceutique pour l'utilisation de la revendication 1, la composition pharmaceutique comprenant en outre au moins un autre type de cellule et/ou au moins un agent.

5. Hydrogel et population de cellules ou composition pharmaceutique pour l'utilisation de l'une quelconque des revendications précédentes, le traitement comprenant l'administration de l'hydrogel et de la population de cellules ou composition pharmaceutique dans un disque intervertébral dégénéré, facultativement dans le noyau pulpeux ou dans l'anneau fibreux du disque intervertébral.

6. Hydrogel et population de cellules ou composition pharmaceutique pour l'utilisation de l'une quelconque des revendications précédentes, comprenant en outre l'induction de la population homogène isolée de cellules obtenue à partir de tissus de cordon ombilical humain pour se différencier au moins partiellement *in vitro,* où facultativement, la population homogène isolée de cellules est induite pour se différencier en cellules présentant un phénotype de cellule d'anneau fibreux ou un phénotype de cellule de noyau pulpeux.

7. Hydrogel et population de cellules pour l'utilisation de l'une quelconque des revendications précédentes, le traitement comprenant l'administration de l'hydrogel simultanément avec, ou avant, ou après, la population homogène isolée de cellules obtenue à partir de tissu de cordon ombilical humain.

8. Hydrogel et population de cellules pour l'utilisation de l'une quelconque des revendications précédentes, le traitement comprenant l'administration de la population homogène isolée de cellules dans un dispositif implantable.

9. Hydrogel et population de cellules pour l'utilisation de l'une quelconque des revendications précédentes, le traitement comprenant en outre l'administration d'au moins un autre type de cellule simultanément avec, ou avant, ou après, la population homogène isolée de cellules obtenue à partir de tissu de cordon ombilical humain.

10. Hydrogel et population de cellules ou composition pharmaceutique pour l'utilisation de la revendication 4 ou 9, l'au moins un autre type de cellule étant modifié de manière à exprimer au moins un produit de gène exogène, le produit de gène exogène étant facultativement un facteur trophique.

11. Hydrogel et population de cellules pour l'utilisation de la revendication 10, le produit de gène exogène modulant l'expression d'une ou plusieurs protéines de matrice extracellulaire.

12. Hydrogel et population de cellules pour l'utilisation de l'une quelconque des revendications précédentes, le traitement comprenant en outre l'administration d'au moins un agent.

13. Hydrogel et population de cellules ou composition pharmaceutique pour l'utilisation de la revendication 4 ou la revendication 12, l'au moins un agent étant un facteur trophique, facultativement choisi dans le groupe constitué de TGF-bêta, GDF-5, PDGF-BB et TIMP1, où, facultativement, le facteur trophique exerce un effet trophique sur la population homogène isolée de cellules obtenue à partir de tissu de cordon ombilical humain.

14. Hydrogel et population de cellules ou composition pharmaceutique pour l'utilisation de la revendication 1, l'hydrogel comprenant le fibrinogène et une colle de thrombine et/ou fibrine.

**A.**

L1-2
L2-3
L3-4
L4-5
L5-6

0wks          6wks          12wks

**B.**

0wks     3wks     6wks     12wks

**C.**

0wks     3wks     6wks     12wks

**Figure 1**

**A.**

0wks  3wks  6wks  12wks

**B.**

0wks  3wks  6wks  12wks

**Figure 2**

A.

B.

Figure 3

Figure 4

**A.**

**B.**

**Figure 5**

A.

B.

Figure 6

**Figure 7**

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 6352557 B, Ferree
- US 6340369 B, Ferree II
- US 6355699 B
- US 5670483 A
- US 5955343 A
- US 20020160471 A
- WO 02062969 A
- US 20020022676 A, He
- US 20080166328 A
- US 7510873 B
- US 7524489 B
- WO 2003025149 A, Ho
- WO 13111933 A
- WO 12337439 A
- WO 61016849 A

## Non-patent literature cited in the description

- **ALINI.** *Eur. Spine J.,* 2002, vol. 11 (2), 215-220
- **TRESCO, P. A. et al.** *Advanced Drug Delivery Reviews,* 2000, vol. 42, 2-37
- **ANSETH, K.S. et al.** *J. Controlled Release,* 2002, vol. 78, 199-209
- **WANG, D. et al.** *Biomaterials,* 2003, vol. 24, 3969-3980
- **MOMBAERTS et al.** *Proc. Nat. Acad. Sci. U.S.A.,* 1991, vol. 88, 3084-3087
- Basic Methods In Molecular Biology. Appleton & Lange, 1994
- **CAMPBELL et al.** *J. Immun.,* 1991, vol. 147 (4), 1238-1246
- **MESSINA et al.** *Exper. Neurol.,* 2003, vol. 184, 816-29
- **TUSHER et al.** *Proc. Natl. Acad. Sci. USA,* 2002, vol. 98, 5116-21
- **ABBAS ; LICHTMAN.** Cellular and Molecular Immunology. Saunders, 2003, 171
- **COUMANS et al.** *Journal of Immunological Methods,* 1999, vol. 224, 185-196
- **BROWN.** *The Journal of Immunology,* 2003, vol. 170, 1257-1266
- **ROSEN et al.** *Ciba Found. Symp.,* 1997, vol. 212, 215-26
- **SALCEDO et al.** *Blood,* 2000, vol. 96, 34-40
- **LI et al.** *J. Immunol.,* 2003, vol. 170, 3369-76
- **HUGHES et al.** *Ann. Thorac. Surg.,* 2004, vol. 77, 812-8
- **CHENG et al.** *Dev. Biol.,* 2003, vol. 258, 319-33
- **CHUJO et al.** *Spine,* 2006, vol. 31, 2909-17
- **MASUDA et al.** *Spine,* 2006, vol. 31, 742-54
- **LU et al.** *Spine,* 1997, vol. 22, 1828-34
- **LIU, K et al.** *PNAS,* 1999, vol. 96, 5147-5152
- **PLAIA et al.** *Stem Cells,* 2006, vol. 24 (3), 531-546
- **SOBAJIMA et al.** *Spine,* 2005, vol. 30 (1), 15-24
- **MASUDA et al.** *Spine,* 2005, vol. 30 (1), 5-14
- **BECHARA et al.** *Am J Neuroradiology,* 2010, vol. 31 (9), 1640-1644
- **WILKE et al.** *Spine,* 1999, vol. 24 (8), 755-762